# EUROPEAN PATENT SPECIFICATION

(11) **EP 2 182 902 B1**
(45) Date of publication and mention of the grant of the patent: **07.01.2015**
(21) Application number: 08797363.2
(22) Date of filing: 07.08.2008
(51) Int. Cl.: A61K 9/70, A61K 31/4535, A61K 9/00, A61K 31/5517, A61P 25/20

(54) **COMPOSITIONS COMPRISING SUFENTANIL AND TRIAZOLAM FOR PROCEDURAL SEDATION AND ANALGESIA USING ORAL TRANSMUCOSAL DOSAGE FORMS**
ZUSAMMENSETZUNGEN MIT SUFENTANIL UND TRIAZOLAM ZUR SEDIERUNG UND ANALGESIE BEI EINGRIFFEN MIT ORALEN TRANSMUKOSALEN DOSIERFORMEN
COMPOSITIONS COMPRENANT DU SUFENTANIL AND DE LA TRIAZOLAME POUR LEUR UTILISATION DANS LA SÉDATION ET L'ANALGÉSIE PROCÉDURALES UTILISANT LES FORMES POSOLOGIQUES TRANSMUCOSALES ORALES

(30) Priority: 07.08.2007 US 954501 P
(43) Date of publication of application: 12.05.2010
(60) Divisional application: 13158781.8
(73) Proprietor: Acelrx Pharmaceuticals, Inc., Redwood City, CA 94063 (US)
(72) Inventor: PALMER, Pamela, San Francisco, California 94127 (US); TZANNIS, Stelios, Newark, California 94560 (US); SCHRECK, Thomas, Portola Valley, California 94028 (US)
(74) Representative: Zwicker, Jörk
(86) International application number: PCT/US2008/072445
(87) International publication number: WO 2009/021106

(56) References cited:
- WO-A2-2007/081949
- WO-A2-2008/085765
- US-A1- 2002 160 043
- US-A1- 2008 166 404
- KRAUSS B ET AL: "Procedural sedation and analgesia in children" LANCET THE, LANCET LIMITED. LONDON, GB LNKD- DOI:10.1016/S0140-6736(06)68230-5, vol. 367, no. 9512, 4 March 2006 (2006-03-04), pages 766-780, XP025094331 ISSN: 0140-6736 [retrieved on 2006-03-04]
- "AcelRx Pharmaceuticals Reports Positive Results from a Clinical Trial of Sublingual Sufentanil/Triazolam NanoTabTM Combination (ARX03) in Treating Procedural Pain and Anxiety" QCELRX PHARMACEUTICALS,, 12 January 2009 (2009-01-12), pages 1-2, XP007914719
- RAZA ET AL.: 'Haemodynamic stability with midazolam-ketamine-sufentanil analgesia in cardiac patients' CAN. J. ANAESTH. vol. 36, no. 6, 1989, pages 617 - 623, XP007914723
- WALDER ET AL.: 'Analgesia and sedation in critically ill patients' SWISS MED. WKLY vol. 134, 2004, pages 333 - 346, XP007914739
- PAVLIN ET AL.: 'Effects of Combining Propofol and Alfentanil on Ventilation, Analgesia, Sedation, and Emesis in Human Volunteers' ANESTHESIOLOGY vol. 84, no. 1, January 1996, pages 23 - 37, XP008131341
- DEMEULES ET AL.: 'Clinical pharmacology and rationale of analgesic combinations' EUROPEAN JOURNAL OF ANAESTHESIOLOGY vol. 20, no. 28 28, 2003, pages 7 - 12, XP007914740

## Description

### BACKGROUND OF THE INVENTION

### Cross Reference to Other Applications

This application claims priority benefit of U.S. Provisional Application Serial No. 60/954,501, filed August 7, 2007.

### Field of the Invention

The present invention relates to compositions, and systems effective to sedate and provide analgesia to a subject during a diagnostic or therapeutic procedure or prior to induction of general anesthesia (procedural sedation and analgesia), comprising the combination of an analgesic drug, such as sufentanil and the drug triazolam typically used to treat anxiety, delivered by the oral transmucosal route in a single dosage form.

### Background of the Technology

Currently, standard regimens for procedural sedation and analgesia have clear limitations with regard to ease of administration, onset of action, efficacy and safety. Routes of administration, formulations and dosage among other attributes contribute to these limitations.

Each drug class has benefits and risks. For instance, elderly patients and children typically require lower doses relative to adult patients and children may experience significant fear and discomfort during medication administration. Some medications are administered orally, while many are administered intravenously (IV). Some medications have slow onset, while other medications exhibit drug interactions and still others have side effects.

Reproducible and effective drug delivery technology represents an area of active research and oral transmucosal drug delivery systems offer numerous advantages relative to conventional dosage forms, which include more comfortable and convenient administration, faster onset, improved efficacy, reduced side effects, and improved patient acceptance. This is particularly relevant to procedural sedation and analgesia.

Opioids are powerful sedatives as well as analgesics that are utilized to treat both acute and chronic pain of moderate to severe intensity. Opioids are also used for procedural sedation and analgesia, as they provide both anxiolysis and analgesia. However, opioids can have respiratory depressive effects if not used appropriately and suffer from a high abuse potential. Opioids have a relatively rapid onset of action when administered either IV or transmucosally.

Benzodiazepines are powerful anxiolytic and amnestic agents, however, when given via the oral route, they can have a delayed and erratic onset, as well as delayed post-procedural recovery (Viitanen *et al.,* 1999). There is no direct analgesic effect of benzodiazepines or most sedatives. As a result, anxiety and agitation can result due to under-treated pain caused by IV cannulation or other procedures. Common side effects with the use of anti-anxiety medications include dry mouth, fatigue, dizziness and headaches. More severe side effects such as memory loss, uncoordinated body movements, confusion, and irregular heartbeat may also result.

Greenblatt O.J, et al., N Engl J Med. 1991 Jun 13;324(24):1691-8, show that benzodiazepines such as triazolam caused a greater degree of sedation and greater impairment of psychomotor performance in healthy elderly persons than in young persons who received the same dose based on a study where 26 healthy young subjects (average age of 30) and 21 healthy elderly subjects (average age of 69) received 125 mcg and 250 mcg of triazolam. On the basis of the results, the authors suggest that the dosage of triazolam for elderly persons should be reduced on average by 50 percent.

Procedural sedation is attempted in many clinical settings using a number of intervention scenarios, which generally include use of benzodiazepines and/or opioids via IV, oral tablets, oral liquids, or transmucosal administration. These methods meet with varying degrees of success with respect to onset of action, duration of action, ease of use, safety and side-effects.

When IV access is not available, often either an oral or intranasal benzodiazepine, such as midazolam, or an intranasal opioid, such as sufentanil, is used for procedural sedation (Karl et al., Anesthesiology; 1992; 76:209-215). There are disadvantages to using a single agent for procedural sedation. There is no direct analgesic effect of benzodiazepines or most sedatives, and using opioids alone to provide procedural sedation and analgesia can result in episodes of respiratory depression as well as post-procedural nausea and vomiting (Friesen and Lockhart, Anesthesiology, 1992; 76:46-51; Karl *et al*., 1992).

There is a continuing, unfilled need for compositions, methods, systems and kits for procedural sedation and analgesia. The present invention addresses this need.

### Summary Of The Invention

The invention provides a single small volume solid dosage form for use in procedural sedation and analgesia:
comprising the combination of from 5 micrograms (µg, mcg) to 50 µg (mcg) of sufentanil and from 100 µg (mcg) to 500 µg (mcg) of triazolam, wherein said solid dosage form is bioadhesive, and adapted to adhere to the oral mucosa until most or all of the drug has been delivered by oral transmucosal administration during the period of drug delivery, wherein said solid dosage form has a volume of less then 100 µl (mcl) and a mass of less than 100 mg.

The solid dosage form has a mass selected from the group consisting of less than 100mg, 90mg, 80mg, 70mg, 60mg, 50mg, 40mg, 30mg, 29mg, 28mg, 27mg, 26mg, 25mg, 24mg, 23mg, 22mg, 21mg, 20mg, 19mg, 18mg, 17mg, 16mg, 15mg, 14mg, 13mg, 12mg, 11mg, 10mg, 9mg, 8mg, 7mg, 6mg and 5mg.

The solid dosage comprises from about 5 micrograms (mcg) to about 50mcg of sufentanil and from about 100mcg to about 500mcg of triazolam.

Oral transmucosal administration, e.g., sublingual administration, of the dosage form to a subject results in one or more of the following (1) a Tₘₐₓ for sufentanil with an overall average coefficient of variation of less than 40%; (2) a total area under the RASS sedation curve (AUCₜₒₜₐₗ) which is greater when the combination of sufentanil and triazolam is administered to the subject as compared to administration of an equivalent dose of sufentanil alone; (3) a mean Tₘₐₓ for sufentanil that is substantially the same when the combination of sufentanil and triazolam is administered to the subject as compared to administration of an equivalent dose of sufentanil alone; (4) a mean Cₘₐₓ for sufentanil that is substantially the same when the combination of sufentanil and triazolam is administered to the subject as compared to administration of an equivalent dose of sufentanil alone; (5) onset of sedation which is evident less than one hour after administration; (6) a duration of sedation of 4 hours or less; (7) a relative AUC₀₋ₗₐₛₜ for sufentanil of greater than 60%, 70% or 80%; and (8) a relative AUC₀₋ₗₐₛₜ for sufentanil with a coefficient of variation of less than 40%.

The solid dosage form comprises an amount of sufentanil effective to induce sedation, but below a dose that induces respiratory depression.

The invention further provides single dose applicators (SDAs), comprising a dosage form as described hereinabove.

In practicing one exemplary method of the invention, the dosage form is administered to a subject during an office or clinic procedure or prior to the induction of general anesthesia wherein the subject or patient is sedated following administration.

### Brief Description of the Figures

Figures 1A and 1B are schematic depictions of an exemplary single dose applicator.
Figures 2A - C provide an illustration of one type of single dose applicator and use thereof in delivering a dosage form to a subject.
Figures 3A - F provide an illustration of six additional single dose applicators.
Figure 4 provides an illustration of a multiple dose dispenser where a plurality of single dose applicators are stored prior to use.
Figures 5A - C provide an illustration of additional single dose applicator and multiple dose applicator embodiments.
Figures 6A - B provide an illustration of two stages of use of one embodiment of a single dose applicator.
Figs. 7A - D are schematic depictions of additional examples of single dose applicators (SDAs).
Figs. 8A - D provide a schematic depiction of a multiple dose dispenser which provides for storage of a plurality of SDAs prior to use, and the use of the SDAs for sublingual administration of a drug dosage form.
Figs. 9A - B are a schematic depiction of an alternative embodiment of an SDA which has a pin lock **167** which must be removed before a tablet can be injected from the SDA, as well as a shroud **29** and a valve **33,** which serve to protect the tablet from saliva ingress when the SDA is inserted into the mouth of a subject.

### DETAILED DESCRIPTION OF THE INVENTION

The invention is based on compositions, and systems that rely on a combination of drugs formulated for oral transmucosal delivery for use in procedural sedation and analgesia.

The present invention provides novel formulations wherein the majority of sufentanil is delivered across the oral mucosa. The dosage forms comprise a combination of drugs, for delivery with or without a device that produce a therapeutic effect and a predictable and safe pharmacokinetic profile.

This is important in the procedural setting, in particular in the non-hospital setting where standard anesthesia cannot be administered safely and effectively. This is also important in both inpatient and outpatient settings when difficult IV access (due to fragile veins, obesity, pediatric patients, etc) necessitates a non-invasive route to relieve the patient's anxiety/pain prior to, or in place of IV cannulation.

In one embodiment, the present invention provides a combination formulation comprised of both the anxiolytic benzodiazepine, triazolam and the fentanyl congener sufentanil.

The following disclosure describes compositions, and systems which find utility in practicing the present invention. The invention is not limited to the specific formulations and methodology or medical conditions described herein, as such may, of course, vary. It is also to be understood that the terminology used herein is for the purpose of describing particular embodiments only, and is not intended to limit the scope of the present invention.

It must be noted that as used herein and in the appended claims, the singular forms "a", "and", and "the" include plural references unless the context clearly dictates otherwise. Thus, for example, reference to "a drug formulation" includes a plurality of such formulations and reference to "a drug delivery device" includes systems comprising drug formulations and devices for containment, storage and delivery of such formulations.
Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

### Definitions

The term "active agent" or "active" may be used interchangeably herein with the term "drug" and is used herein to refer to any therapeutically active agent.

As used herein, when a drug formulation is said to "adhere" to a surface, such as a mucosal membrane, it is meant that the formulation is in contact with said surface and is retained on the surface without the application of an external force. Adhesion is not meant to imply any particular degree of sticking or bonding, nor is it meant to imply any degree of permanency.

As used herein, the term "analgesic", is used with reference to any of a number of drugs used to relieve pain (achieve analgesia).

The term "AUC" as used herein means "area under the curve" in a plot of concentration of drug in plasma versus time. AUC is usually given for the time interval zero to infinity, however, clearly plasma drug concentrations cannot be measured 'to infinity' for a patient so mathematical approaches are used to estimate the AUC from a limited number of concentration measurements.

The term "AUC_{0-inf}" as used herein means, the AUC (from zero to infinity) and represents the total amount of drug absorbed by the body, irrespective of the rate of absorption. The AUC of a transmucosal dosage form compared to that of the same dosage administered intravenously serves as the basis for a measurement of bioavailability.

The term "AUC₀₋ₗₐₛₜ" is used herein with reference to the AUC (from zero to last measurement).

The term "relative AUC₀₋ₗₐₛₜ" is used herein with reference to the AUC₀₋ₗₐₛₜ of the test article following delivery via the intended route versus the AUC₀₋ₗₐₛₜ for the same drug after intravenous (sufentanil) or oral (triazolam) administration.

The term "AUCₜₒₜₐₗ" as used herein with respect to sedation means "area under the curve" in a plot of the results from the Richmond Agitation Sedation Scale (RASS) versus time for the time period from administration of a drug dosage form (time 0) following administration to the last time-point of RASS analysis at 640 minutes.

The term "anxiolytic" as used herein refers to a drug prescribed for the treatment of symptoms of anxiety.

The term "bioadhesion" as used herein refers to the process of adhesion of the dosage forms to a biological surface, e.g., a mucosal membrane.

The term "bioavailability" or "F" as used herein means "percent bioavailability" and represents the fraction of drug absorbed from a test article as compared to the same drug when administered intravenously. It is calculated from the AUC_{∞} of the test article following delivery via the intended route versus the AUC_{∞} for the same drug after intravenous administration. It is calculated from the equation: Bioavailability (%) = AUC_{∞} (test article)/ AUC_{∞} (intravenous route/article).

The term "congener" as used herein refers to one of many variants or configurations of a common chemical structure.

"Controlled drug delivery" refers to release or administration of a drug from a given dosage form in a controlled fashion in order to achieve the desired pharmacokinetic profile *in vivo.* An aspect of "controlled" drug delivery is the ability to manipulate the formulation and/or dosage form in order to establish the desired kinetics of drug release.

The term "disintegration" is used interchangeably herein with "erosion" and means the physical process by which a dosage form breaks down and pertains to the physical integrity of the dosage form alone. This can occur in a number of different ways including breaking into smaller pieces and ultimately, fine and large particulates or, alternatively, eroding from the outside in, until the dosage form has disappeared.

The term "formulation" or "drug formulation" or "dosage form" as used herein refers to a composition containing at least one therapeutic agent or medication for delivery to a subject. The dosage form comprises a given "formulation" or "drug formulation" and may be administered to a patient in the form of a lozenge, pill, tablet, capsule, membrane, strip, liquid, patch, film, gel, spray or other form.

The terms "drug", "medication", "pharmacologically active agent", "therapeutic agent" and the like are used interchangeably herein and generally refer to any substance that alters the physiology of an animal. A dosage from comprising a formulation may be used to deliver any drug that may be administered by the oral transmucosal route. "Drug" as used herein with reference to a formulation of the invention means any "drug"," active agent", "active", "medication" or "therapeutically active agent" that can be effectively administered by the oral transmucosal route. It will be understood that a "drug" formulation of the invention may include more than one therapeutic agent, wherein exemplary combinations of therapeutic agents include a combination of an opioid analogue, such as sufentanil, fentanyl, alfentanil, lofentanil, carfentanil, remifentanil, trefentanil, or mirfentanil, in combination with a drug typically used for the treatment of anxiety.

The expression "mucoadhesion" is used herein to refer to adhesion to a membrane which is covered by mucus, such as those in the oral cavity. The term "mucoadhesion" may be used interchangeably herein with the term "bioadhesion".

The term "mucosal membrane" refers generally to any of the mucus-coated biological membranes in the body. Thus, oral mucosal absorption, i.e., buccal, sublingual, gingival and palatal absorption are specifically contemplated.

The term "procedural sedation and analgesia" is used herein with reference to producing a state of relaxation or sleepiness and a state of decreased pain during a diagnostic or therapeutic procedure or prior to the induction of general anesthesia in a subject or patient by administration of one or more drugs. Sedation may be conscious or unconscious depending on the dose of drug delivered and the age and weight of the patient or subject. Conscious sedation does not alter respiratory, cardiac, or reflex functions to the level that requires external support for these vital functions. Unconscious sedation is a controlled state of anesthesia, characterized by partial or complete loss of protective nerve reflexes, including the ability to independently breathe and respond to commands.

The term "subject" includes any subject, generally a mammal (e.g., human, canine, feline, equine, bovine, ungulate etc.), adult or child, in which treatment for a disorder is desired. The terms "subject" and "patient" may be used interchangeably herein.

The term "oral transmucosal dosage form" is used with reference to a dosage form, which comprises a drug formulation as described herein. The oral dosage form is used to deliver a pharmaceutically active substance to the circulation by way of the oral mucosa and is typically a "sublingual dosage form" or "buccal dosage form", however, in some cases other oral transmucosal routes may be employed. The dosage form provides for delivery of pharmaceutically active substances across the oral mucosa and by controlling the formulation the timing for release of the pharmaceutically active substance can be achieved. The dosage form comprises pharmaceutically acceptable excipients and the drug formulations which comprise the dosage form are neither effervescent nor do they comprise an essentially water-free, ordered mixture of microparticles of drug adhered to the surface of carrier particles, where the carrier particles are substantially larger than the microparticles of drug.

The term "oral transmucosal drug delivery" as used herein refers to a dosage form wherein drug delivery occurs substantially via the oral transmucosal route and not via swallowing followed by GI absorption. The formulations and drug dosage forms are designed to provide for a drug dissolution rate and dosage form erosion rate that allows for maximal delivery via the oral mucosa, typically via placement of the dosage form within the sublingual cavity.

The term "sedation" as used herein with respect to the administration of sedative drugs, generally to facilitate a medical procedure. Sedation is evaluated using a number of tests, one example of which is the Richmond Agitation Sedation Scale (RASS). If the RASS score of a subject is less than 0 at a given point in time, the subject is considered to be "sedated" at that time. The RASS scale is described in the literature, e.g., in Sessler, et al., American Journal of Respiratory and Critical Care Medicine Vol 166. pp. 1338-1344, (2002).

The term "small volume drug dosage form" or "small volume dosage form" is used herein with reference to a small volume dosage form that has a volume of less than 100mcl and a mass of less than 100mg. More specifically, the dosage form has a mass of less than 100mg, 90mg, 80mg, 70mg, 60mg, 50mg, 40mg, 30mg, 29mg, 28mg, 27mg, 26mg, 25mg, 24mg, 23mg, 22mg, 21mg, 20mg, 19mg, 18mg, 17mg, 16mg, 15mg, 14mg, 13mg, 12mg, 11mg, 10mg, 9mg, 8mg, 7mg, 6mg or 5mg or a volume of less than 100mcl, 90mcl, 80mcl, 70mcl, 60 mcl, 50mcl, 40mcl, 30mcl, 29 mcl, 28 mcl, 27mcl, 26mcl, 25mcl, 24mcl, 23mcl, 22mcl, 21mcl, 20mcl, 19mcl, 18mcl, 17mcl, 16mcl, 15mcl, 14mcl, 13mcl, 12mcl, 11mcl, 10mcl, 9mcl, 8mcl, 7mcl, 6mcl or 5mcl. The "dosage form" may or may not have bioadhesive characteristics and may form a hydrogel upon contact with an aqueous solution. The "small volume drug dosage form" or "small volume dosage form may be referred to as a "NanoTab™".

As used herein, "sublingual", means literally "under the tongue" and refers to a method of administering substances via the mouth in such a way that the substances are rapidly absorbed via the blood vessels under the tongue rather than via the digestive tract. Absorption occurs via highly vascularized sublingual mucosa and allows a substance more direct access to the blood circulation, providing for direct systemic administration independent of gastro-intestinal influences.

The term "transmucosal" delivery of a drug and the like is meant to encompass all forms of delivery across or through a mucosal membrane. In particular," oral transmucosal" delivery of a drug includes delivery across any tissue of the mouth, pharynx, larynx, trachea, or upper gastrointestinal tract, particularly the sublingual, gingival and palatal mucosal tissues.

The term "therapeutically effective amount" means an amount of a therapeutic agent, or a rate of delivery of a therapeutic agent (e.g., amount over time), effective to facilitate a desired therapeutic effect, such as pain relief. The precise desired therapeutic effect (e.g., the degree of pain relief, and source of the pain relieved, etc.) will vary according to the condition to be treated, the tolerance of the subject, the drug and/or drug formulation to be administered (e.g., the potency of the therapeutic agent (drug), the concentration of drug in the formulation, and the like), and a variety of other factors that are appreciated by those of ordinary skill in the art.

The term "Tₘₐₓ" as used herein means the time point of maximum observed plasma concentration.

The term "Cₘₐₓ" as used herein means the maximum observed plasma concentration following administration of a drug.

The term "terminal half-life" or "t½ [h]" as defined herein is calculated as In(2)/λz (defined as the first order terminal rate constant estimated by linear regression of the time versus log concentration curve) and also determined after the final dosing in repeated dose studies.

The term "Tₒₙₛₑₜ" with respect to sedation is used herein relative to the observed "time of onset" and represents the time required for the RASS score to become less than zero for the first time.

### DESCRIPTION OF EXEMPLARY EMBODIMENTS

The present invention is directed to compositions, and systems for procedural sedation and analgesia.

The invention relies on small oral transmucosal dosage forms comprising formulations effective for induction of procedural sedation and analgesia, for example prior to a therapeutic procedure or prior to induction of general anesthesia. The dosage forms comprise the combination of triazolam typically used to treat anxiety, and the analgesic drug; sufentanil, delivered by the oral transmucosal route in a single dosage form.

In one exemplary application, the invention finds utility both in clinics, doctor's offices, and in the hospital setting for use in place of oral or IV drugs in order to effect procedural sedation and analgesia. This is particularly important for populations such as pediatric patients, obese patients, elderly patients with fragile veins, patients with cancer undergoing chemotherapy, and the like.

### Benzodiazepines

Benzodiazepines are drugs that relieve anxiety putatively by acting on the limbic system, an area deep inside the brain that appears to be involved in primitive emotional responses. Exemplary drugs of the benzodiazepine class include but are not limited to triazolam, midazolam, temazepam, estazolam, alprazolam, diazepam and lorazepam, and are usually taken orally.

Oral benzodiazepines act fairly rapidly (within 1-2 hours), with a limited number of side effects which can include agitation, worsened anxiety, confusion, impaired memory, lack of coordination, speech difficulties, and others.

Some patients, in particular those who have had problems with alcohol or drug dependency, may become dependent on the chronic use of benzodiazepines, however, very short-term, acute use of benzodiazepines, for procedural sedation for example, has not been shown to lead to physical dependence and addiction. Using the sublingual route to deliver benzodiazepines pre-procedurally has resulted in effective sedation as demonstrated by the studies referenced below.

### Triazolam

Triazolam or 8-chloro-6-(o-chlorophenyl) -1-methyl-4H-s-triazolo-[4,3-alpha][1,4]benzodiazepine has a molecular weight of 343 and is marketed under brand names Halcion®, Novodorm®, Songar®). Triazolam is a benzodiazepine derivative that is generally only used as a sedative to treat insomnia.

Orally administered triazolam has a plasma half-life of 1.5 - 5.5 hours, the shortest of the clinically used benzodiazepines. Studies comparing pharmacokinetics of triazolam demonstrated a 50% increase in Cₘₐₓ but no change in Tₘₐₓ (0.9 hours) for elderly versus young adults. The clearance of triazolam in the elderly was approximately 40% less than young adults. Triazolam is currently approved for the short-term treatment of insomnia (generally 7 - 10 days). Triazolam is available as an oral tablet at two dosage strengths: 0.125 mg and 0.250 mg. A 0.2 mg sublingual triazolam tablet was marketed as Dumozolam®, by Dumex Ltd., Denmark, however, it is no longer commercially available. While oral triazolam is usually used as a sleeping aid for patients with insomnia, there are also studies demonstrating the successful use of this medication for procedural anxiety. Comparison of the pharmacokinetics of sublingual triazolam with oral administration demonstrates a 28% higher bioavailability and a 20% higher peak plasma level for the sublingual route of administration. The effects of triazolam are reversed by administration of flumazenil. The initial step in triazolam metabolism is hydroxylation catalyzed by cytochrome **P450 3A (CYP 3A).**

Sublingual administration of triazolam has been described as effective for preoperative sedation in a number of situations: (1) Sublingual administration of 250 mcg of triazolam for preoperative sedation 60 minutes prior to oral surgery in dental outpatients resulted in significantly less anxiety and pain at 15 minutes intraoperatively than both oral triazolam and placebo. The observed decrease in pain may have been an indirect effect since benzodiazepines have been shown to not possess direct analgesic. Comparison of the pharmacokinetics of sublingual triazolam with oral administration demonstrated a 28% higher bioavailability and a higher peak plasma level for the sublingual route of administration. Tablets were the size of 325 mg acetaminophen and dissolved within 90 seconds. Tₘₐₓ for both oral and sublingual sufentanil was approximately 90 minutes. (Berthold CW, et al., Oral Surg Oral Med Oral Pathol Oral Radiol Endod; 1997; 84(2):119-24); (2) the PK of triazolam was evaluated in 9 healthy children, aged 6 to 9 years, who received oral triazolam (0.025 mg/kg suspended in Kool-Aid) before dental treatment. The peak plasma concentration was 8.5 +/- 3.0 ng/mL (mean +/- SO). The time to peak plasma concentration was 74 +/- 25 minutes. Recovery from sedation required 180 to 240 minutes (Karl H. W, et al., Journal Clinical Psychopharmacology; 1997; 17(3):169-172); (3) the clinical effects of a 200 mcg sublingual triazolam tablet were compared with those of a 10 mg tablet of diazepam in a double-blind study, in 100 61-70 year old patients scheduled for ophthalmic surgery under local anesthesia. Surgery began at least 45 minutes after administration of triazolam. Authors concluded that sedation developed 60-90 minutes after administration of 200 mcg sublingual triazolam, that 200 mcg sublingual triazolam produced deeper sedation than 10 mg oral diazepam. (Kontinen V, et al., Canadian Journal of Anesthesia, Vol 40, 829-834, 1993); (4) the relative and absolute bioavailability of triazolam was evaluated after administration by the oral and sublingual routes. The fraction absorbed relative to intravenous was 20% higher in the sublingual than in the oral treatment (p = 0.0128); the difference between treatments was greatest in the first 2 hours as indicated by the area under the curve from 0 to 2 hours (p < 0.05) describe the relative and absolute bioavailability of triazolam evaluated after administration of the marketed oral tablet (250 mcg Halcion) and a sublingual prototype wafer with an IV comparator in 12 men. The fraction absorbed relative to intravenous was 20% higher in the sublingual than in the oral treatment (p = 0.0128); the difference between treatments was greatest in the first 2 hours as indicated by the area under the curve from 0 to 2 hours (p < 0.05). Tₘₐₓ for sublingual triazolam was approximately 1.19 hours (71.4 minutes) (Kroboth PD et al., J Clin Psychopharmacol; 1995; 15(4):259-62); (5) eight healthy adult volunteers received 500mcg of triazolam in a commercially available tablet by sublingual and oral routes on two occasions in random sequence. The bioavailability of triazolam after sublingual administration was shown to be an average of 28% greater than for oral administration of the same dose. The mean total area under the curve for sublingual administration was significantly larger than that following oral dosage (28.9 vs 22.6 ng-hr/mL, p < 0.025). The peak plasma concentration after sublingual dosage was also higher than after oral administration (4.7 vs 3.9 ng/mL, p < 0.1). No significant differences between sublingual and oral administration were found for the elimination half-life of triazolam (4.1 vs 3.7 hr) and the time of peak concentration (1.22 vs 1.25 hr) after dose. (Scavone JM, et al., J Clin Pharmacol; 1986; 26:208-10); (6) a study on the pharmacokinetics of sublingual triazolam in children, where nine healthy children (64-98 months old) received 250 mcg or 375 mcg of sublingual triazolam before dental treatment indicated a Cₘₐₓ of 4.9 +/- 2.0 ng/mL (mean +/- SD) with a range of 4.0-8.2 ng/ml, a Tₘₐₓ of 75 +/- 32 minutes with a range of 30-120 minutes, and an elimination half-life of 91 +/- 32 minutes with a range of 51-140 minutes. Average sublingual tablet dissolution was 4 minutes (Tweedy et al., J Clin Psychopharmacol. 2001, 21(3):268-72); (7) a review of dental literature suggests that the oral and sublingual dose range for producing sedation is 250-500mcg and that it is effective when administered 30-45 minutes before a procedure. In a study where 10 healthy adult volunteers (18-40) received sublingual triazolam (250 mcg Halcion) followed by additional doses after 60 (500 mcg) and 90 (250 mcg) minutes, Cₘₐₓ was greater than 90 minutes after the last dose and thus was not determined. Tablets dissolved in 2-3 minutes after dosing. (Jackson D, et al., Journal Clinical Psychopharmacology; 2006; 26(1):4-8).

### Midazolam

Oral midazolam is used as a sedative before or during surgery or a medical procedure. Midazolam is very fast acting and therefore useful for anesthesia because it produces sedation, amnesia, and relief of anxiety. It has become a commonly used agent for conscious sedation of children before diagnostic or therapeutic procedures and before induction of anesthesia.

Midazolam or 8-chloro-6-(2-fluorophenyl)-1-methyl-4H-imidazo[1,5-a][1,4]benzodiazepine has a molecular weight of 326. Midazolam is marketed under brand names Dormicum, Flormidal, Versed, Hypnovel and Dormonid and is a benzodiazepine derivative. It has powerful anxiolytic, amnestic, hypnotic, anticonvulsant, skeletal muscle relaxant and sedative properties. It is considered a fast-acting benzodiazepine, with a short elimination half-life. Midazolam has an oral bioavailability of approximately 36% (with a broad range) and orally administered midazolam has a plasma half-life of 1.5 - 5 hours. In adults greater than 60 years, the plasma half-life of midazolam may be prolonged up to 3 times. The pharmacokinetics of midazolam is linear in the 7.5-15 mg oral dose range. Midazolam is absorbed rapidly and completely after oral administration. With a dose of 15 mg, maximum plasma concentrations of 70-120 ng/ ml are reached within one hour. Food prolongs the time to peak plasma concentration.

Until recently, only an intravenous form of the drug was available and medical and dental practitioners typically used the intravenous form for oral administration to avoid the additional trauma of starting an IV in children. However, the liquid was bitter even with added flavoring. In November 1998, the food and Drug Administration approved a clear, purplish-red, cherry flavored midazolam-containing liquid that contains an artificial bitterness modifier (Versed Syrup), and 2 mg midazolam per 1 ml. The reported acceptance rate by children was 90%. The recommended dose for children is a single dose of 0.25 to 0.5 mg/kg to a maximum dose of 20 mg. The most serious side effect of midazolam is respiratory depression or arrest, which can be reversed by flumazenil (Romazicon).

The sedative effects of sublingual midazolam (Roche, Dormicum, 7.5 mg) with the oral route as a premedication were compared. There were 50 patients in each group, the degree of sedation was assessed and the time for complete drug dissolution studied in the sublingual group by the inspection of tablet under the tongue every 5 minutes for 20 min. The sedation scores in the sublingual group were higher than in the oral group at 30 and 60 min after drug administration. 72% of the sublingual group had complete drug dissolution within 10 min and 64% of the patients in the sublingual group found the tablet acceptable with regard to taste (Lim et al., Can J Anaesth; 1997; 44(7):723-6).

Transmucosal administration of midazolam has been described as effective for preoperative sedation in a number of situations: (1) midazolam was administered transmucosally in 47 children randomly assigned to 3 different groups. Group N received 0.2 mg/kg nasally, group R 0.5 mg/kg rectally, and group S 0.2 mg/kg sublingually. 30 min after premedication the midazolam level in the sublingual group was statistically significantly higher than in the nasal group. (Geldner G, et al., Paediatric Anaesthesia; 1997 (7):103-109); (2) nasal midazolam was shown to be effective in the treatment of acute seizures (Jeannet Pet al., Eur J of Paediatric Neurology, 1999, 3:73-77); (3) in a prospective, double-blind, placebo-controlled trial, children scheduled for day surgery received either injectable midazolam mixed with a thick grape syrup and placed under the tongue in one of 3 doses (0.25, 0.5, or 0.75 mg/kg) or placebo and children readily accepted the mixture. None of the children receiving placebo, 28% receiving 0.25 mg/kg (P = 0.02), 52% receiving 0.5 mg/kg (P < 0.001), and 64% receiving 0.75 mg/kg (P < 0.001) of midazolam showed satisfactory sedation at 15 min after administration (Khalil et al., Paediatric Anaesthesia, 1998; (8):461-465); (4) 60 children received either oral midazolam 0.5 mg/kg or placebo approximately 30 min before the induction of anesthesia and the authors concluded that benzodiazepines, especially when given via the oral route, can have a delayed and erratic onset which results in delayed post-procedural recovery (Viitanen H, et al., Can J Anesth., 1999, 46(8):766-771); (5) when intranasal midazolam was compared with sufentanil as a premedicant for 60 pediatric patients, aged 1/2 to 6 years, undergoing outpatient surgery of 2 hours or less, children who had not previously cried were more likely to cry when midazolam was administered compared with sufentanil (71% versus 20%, p = 0.0031), and of 31 midazolam patients, 20 experienced nasal irritation. (Zedie N, et al., Clin Pharmacol Ther; 1996; 59:341-8); and (6) a review article by McCann and Kain (Anesthesia & Analgesia, 93:98-105, 2001) reports that although transmucosal benzodiazepines, such as midazolam, have a rapid onset of action, the intranasal route is irritating and creates crying episodes and the sublingual route results in swallowing or spitting out of the drug.

### Anxiety

Anxiety is a complex feeling of apprehension, fear, and worry often accompanied by pulmonary, cardiac, and other physical sensations. It is a common condition that can be a self-limited physiologic response to a stressor, or it can persist and result in debilitating emotions.

Anxiety may surround a specific condition or situation, such as an intense fear prior to a medical or dental procedure. The fear of a subject may be so severe that they may experience physical symptoms of anxiety, and even have panic attacks, when confronted with the situation, or even anticipating having to deal with the situation.

A subject may either avoid having a medical or dental procedure they fear or endure the situation with distress. This is particularly problematic in the pediatric situation as children often do not know that their fear of a situation is excessive or unreasonable.

Physicians and nurses are often required to perform procedures on children and adults that are perceived as painful or frightening. Children often view needle sticks as a source of pain and fear. In an effort to minimize the pain of needle sticks, the use of a mixture of lidocaine and prilocaine (EMLA) has become standard practice in many children's hospitals. Unfortunately, EMLA requires at least 60 minutes to be fully effective and reportedly may cause vasoconstriction, leading to difficult vein cannulation.

Procedural anxiety and successful sedation have been inversely correlated. It has been shown that children with low anxiety are 3.8 times more likely to be successfully sedated (Schreiber KM et al., Am J Emerg Med. 2006 Jul;24(4):397-401).

Patients scheduled for a variety of office or clinic procedures are often anxious and frightened. High levels of anxiety may result in more difficult and painful procedures. Some exemplary procedures include breast core-needle biopsy, dental procedures, cosmetic procedures, dermatologic procedures, podiatric procedures, setting broken bones or spinal injections, among others.

A number of classes of drugs are used to treat anxiety, including but not limited to, benzodiazepines, beta blockers, miscellaneous anxiolytics, monoamine oxidase inhibitors, selective serotonin reuptake inhibitors (SSRIs), serotonin-norepinephrine reuptake inhibitors (SNRIs) and tricyclic antidepressants. Certain drug classes have greater effectiveness for specific anxiety disorders than others.

For an acute anxiety attack, short-term treatment with benzodiazepines is a standard treatment. More chronic episodes of anxiety are typically treated by administration of SSRIs, SNRIs or buspirone. In other situations, tricyclic antidepressants, beta-blockers, and, rarely, monoamine oxidase inhibitors are prescribed alone or in combination with other drugs to control anxiety.

### Sufentanil and Other Opioids

Opioids are powerful analgesics and are utilized to treat both acute and chronic pain of moderate to severe intensity. Transmucosal administration of opioids has been used to treat procedural anxiety, especially in children, however, the dose required for sedation using an opioid alone is higher than required for analgesic purposes and may result in an increased incidence of respiratory depression and nausea and vomiting, which raises safety concerns and can delay discharge from the post-surgical recovery room (Clin. Pharmacol and Therapeutics 59:341, 1996).

Sufentanil (N-[(4-(Methoxymethyl-1-(2-(2-thienyl)ethyl)-4-piperidinyl)]-N-phenylpropanamide), is used as a primary anesthetic, to produce balanced general anesthesia in cardiac surgery, for epidural administration during labor and delivery and has been administered experimentally in both intranasal and liquid oral formulations. A commercial form of sufentanil used for IV delivery is the SUFENTA FORTE^{®} formulation. This liquid formulation contains 0.075 mg/ml sufentanil citrate (equivalent to 0.05 mg of sufentanil base) and 9.0 mg/ml sodium chloride in water. It has a plasma elimination half-life of 148 minutes, and 80% of the administered dose is excreted in 24 hours. The term sufentanil, as used herein includes sufentanil base, sufentanil citrate or a pharmaceutically acceptable salt or derivative thereof.

The use of sufentanil clinically has predominantly been limited to IV administration in operating rooms or intensive care units. Intranasal sufentanil liquid has been studied in both adult and pediatric patients for procedural sedation, with doses of 5 - 20 mcg or higher providing sedative effects (Vercauteren et al., 1988; Karl et al., 1992). There are some issues relating to slower onset of action and decreased bioavailability, when the medication is inadvertently swallowed. For example, Helmers et al. 1989, describes a double-blind study which compared the efficacy of 15 mcg sufentanil (intranasal vs. IV) for postoperative analgesia, based upon a numeric rating scale (NRS) from 0 to 10 for pain in 16 patients. For intranasal sufentanil liquid, Tₘₐₓ was 10 minutes with a bioavailability of 78% and a peak sedation at 40 minutes. Gardner-Nix J., J Pain Symptom Management. 2001 Aug; 22(2):627-30 describes administration of liquid sublingual sufentanil to adults wherein there was an analgesic effect following administration and that the analgesic onset occurred within 6 minutes with a duration of pain relief of approximately 30 minutes. Vercauteren M et al., Anaesthesia; 1988; 43:270-273, describe effects of intranasal sufentanil liquid in both adult and pediatric patients for procedural sedation, with doses of 10 and 20 mcg or higher providing sedative effects (5 mcg was not sufficient). Onset of sedation was achieved in a median of 10 minutes (range 5-30 minutes) and in 5/40 patients sedation was still evident at 60 minutes. The average duration was 40.8 minutes (range 10-55 minutes).

Prior to the work of the current inventors, no pharmacokinetic data had been published on sublingual sufentanil in any form. Example 1 (below) and United States Patent Publication Nos. 20070207207; 20080166404; and 20080147044; United States Patent Application Serial Nos. 11/650,174 and 11/985,162; and PCT Publication Number WO 2007/081949, describe results from human clinical studies where sufentanil was administered via the oral transmucosal route.

Fentanyl (N-(1-phenethyl-4-piperidyl)-N-phenyl-propanamide) was first synthesized in Belgium in the late 1950s, and has an analgesic potency of about 80 times that of morphine. Fentanyl and its congeners are mu opioid agonists that were originally developed as anesthesia agents, and are often administered intravenously due to rapid onset of analgesia. Fentanyl and other opioid agonists, have the potential for deleterious side effects including respiratory depression, nausea, vomiting and constipation.

Alfentanil, remifentanil, lofentanil, carfentanil, trefentanil, and mirfentanil are also potent fentanyl congeners that are rapidly metabolized and may be suitable for use in a transmucosal formulation in combination with an anxiolytic, such as triazolam.

Following transbuccal administration of fentanyl using a lozenge (e.g., Actiq^{®}), the bioavailability is 50%, although the Tₘₐₓ for the 200 mcg dosage of Actiq^{®} ranges from 20 - 120 minutes resulting from erratic GI uptake due to the fact that 75% of the fentanyl is swallowed (Actiq^{®} package insert). More recent publications on the Tₘₐₓ of Actiq indicate that these original times were skewed towards more rapid onset (Fentora package insert indicates a range of Tₘₐₓ for Actiq extending up to 240 minutes). Fentora (a fentanyl buccal tablet) exhibits a bioavailability of 65%, with reported swallowing of 50% of the drug. In contrast to the claimed dosage forms, both Actiq^{®} and Fentora suffer from the disadvantage that substantial amounts of lozenge-administered fentanyl are swallowed by the patient. Since fentanyl has a 30% bioavailability from the GI route, this swallowed drug can contribute to the Cₘₐₓ plasma levels to a significant degree and results in the erratic Cₘₐₓ and Tₘₐₓ observed with these products.

Oral transmucosal fentanyl lozenge-on-a-stick (Oralet^{®}) was studied for use as a procedural sedative and analgesic in pediatric patients undergoing central venous line removal (Wheeler et al., 2002). The onset of action was both delayed and erratic {Tₘₐₓ = 53 ± 40 minutes) and it was concluded that this fentanyl lozenge was not adequate for procedural sedation in children.

There remains a need for oral transmucosal preparations that are effective sedative agents that can also provide analgesia, which do not result in inadvertent swallowing of the drug due to large saliva responses or nasal run-off.

### Use of Opioids and Other Analgesics For Procedural Sedation and Analgesia.

Although opioids are powerful analgesics as well as sedatives, they are known to produce pruritis, respiratory depression and/or nausea and vomiting during acute use and physical dependence, possible addictive behaviors and tolerance with long-term use. Benzodiazepines are powerful anxiolytics, however they have no analgesic properties.

When IV access is not available, often either a benzodiazepine, such as oral or intranasal midazolam, or an opioid, such as intranasal sufentanil, is used for procedural sedation (Karl et al., Anesthesiology, 76:209-15, 1992). There are disadvantages of using a single agent for procedural sedation. Benzodiazepines, in particular when given via the oral route, can have a delayed and erratic onset which results in delayed post-procedural recovery (Viitanen et al., Anesthesia & Analgesia, 89:75-9, 1999; Viitanen et al, Canadian Journal of Anaesthesia, 46:766-71, 1999).

Sedation coupled with the need for pain relief is necessary in many outpatient settings, such as prior to a potentially painful medical or dental procedure. There is clearly a need for a rapid-acting dosage form that produces effective sedation and relief from anxiety and pain, and which may be used safely and conveniently in the procedural setting.

The combination of the opioid sufentanil and the benzodiazepine triazolam in a single dosage form provides an opportunity to develop a small oral transmucosal dosage providing a non-invasive approach to procedural sedation and analgesia.

As further described herein, there is no direct analgesic effect of benzodiazepines or most sedatives, which can result in increased anxiety and agitation due to under-treated pain. Furthermore, multiple studies have demonstrated delays in post-operative discharge when large doses of oral midazolam are used as a premedication. On the other hand, treatment with opioids alone to provide procedural sedation and analgesia can result in episodes of respiratory depression and post-procedural nausea and vomiting (Friesen and Lockhart, Anesthesiology, 76:46-51, 1992; Karl et al., Anesthesiology, 76:209-15, 1992). Therefore, there are significant advantages for procedural sedation and analgesia in combining both the sedative agent, triazolam with the analgesic agent, sufentanil, in a dosage form that results in high bioavailability with consistent onset and offset of action.

The novel formulations described herein are provided in a single oral transmucosal dosage form that is relatively undetectable due to the small size of the dosage form. The oral transmucosal administration of the combination of the fentanyl congener sufentanil and the benzodiazepine, as triazolam, allows for the dose of each drug to be lowered while effectively sedating the subject.

One exemplary use of the claimed drug dosage forms is to effect sedation and analgesia prior to and during a medical or dental procedure. When the claimed drug dosage forms are used for procedural sedation and analgesia, the opioid agent in the drug dosage form is sufentanil provided in combination with triazolam. Sufentanil may be provided in the claimed dosage forms in any of a number of formulations and forms, e.g., as sufentanil citrate or as sufentanil base.

Yet another preferred embodiment relies on a combination of sufentanil and at least one additional agent typical used for treatment of analgesia, e.g., a combination of sufentanil and alfentanil. Various opioid drugs have different pharmacokinetic profiles and different interactions with mu opioid receptor splice variants and, therefore, may be used in combination to enhance the therapeutic effect.

Preferred dosage forms for use in procedural sedation and analgesia contain from about 5 to about 50mcg of sufentanil per dosage form for oral transmucosal delivery, in combination with triazolam. Dosage form contains from about 5 to about 50mcg of sufentanil in combination with about 100 to about 500mcg of triazolam.

As will be understood by those of skill in the art, the dose will be on the lower end of the range for children and adults over 60 and on the higher end of the range for adults from 18 to 60 years of age, dependent upon body mass, in particular when administered long-term to opioid-tolerant adults.

### Drug Dosage Forms.

The small volume oral transmucosal drug dosage forms described herein produce a reduced saliva response as compared with conventional, larger oral dosage forms that are intended to be swallowed following administration to the oral cavity. Such conventional, larger oral dosage forms often result in a substantial amount of the drug delivered via the gastrointestinal route.

The claimed dosage forms contain a mixture of an opioid, such as sufentanil and a benzodiazepine such as triazolam and provide for high absorption rates of the pharmaceutically active substance across the oral mucosa and reduced uptake via the gastrointestinal tract, thereby offering a more consistent and reproducible pharmacokinetic and corresponding pharmacodynamic profile.

The dosage forms are typically "sublingual dosage forms", but in some cases another oral transmucosal route, such as the buccal route may be employed. The preferred site for oral transmucosal drug delivery is the sublingual area, although in certain embodiments it may be advantageous for the dosage form to be placed inside the cheek, or to adhere to the roof of the mouth or the gum.

Typically, the dosage forms are adapted to adhere to the oral mucosa (i.e. are bioadhesive) during the period of drug delivery, and until most or all of the drug has been delivered from the dosage form to the oral mucosa.

The claimed dosage forms have a mass of less than 100mg or a volume of less than 100 mcl. More specifically, the dosage forms have a mass of less than 100mg, 90mg, 80mg, 70mg, 60mg, 50mg, 40mg, 30mg, 29mg, 28mg, 27mg, 26mg, 25mg, 24mg, 23mg, 22mg, 21mg, 20mg, 19mg, 18mg, 17mg, 16mg, 15mg, 14mg, 13mg, 12mg, 11mg, 10mg, 9mg, 8mg, 7mg, 6mg or 5mg or a volume of less than 100mcl, 90mcl, 80mcl, 70mcl, 60 mcl, 50mcl, 40mcl, 30mcl, 29mg, 28mg, 27mcl, 26mcl, 25mcl, 24mcl, 23mcl, 22mcl, 21mcl, 20mcl, 19mcl, 18mcl, 17mcl, 16mcl, 15mcl, 14mcl, 13mcl, 12mcl, 11mcl, 10mcl, 9mcl, 8mcl, 7mcl, 6mcl or 5mcl. The dosage forms typically have bioadhesive characteristics and may form a hydrogel upon contact with an aqueous solution.

The dosage forms typically have an erosion time of from 30 seconds up to a time selected from the group consisting of 5, 6, 7, 8, 9, 10, 11, 12, 13, 14, 15, 20, 25 and 30 minutes. Preferred dosage forms have an erosion time of less than 6 minutes, and more preferably less than 2 minutes.

In general, at least 55%, at least 60%, at least 65%, at least 70%, at least 75%, at least 80%, at least 85%, at least 90%, at least 95%, at least 98% or at least 99% of the total amount of sufentanil in a dosage form administered to the oral mucosa of a subject is absorbed via the oral transmucosal route.

The dosage forms may have essentially any shape, examples of which include a round disc with a flat, concave, or convex face, an ellipsoid shape, a spherical shape, a polygon with three or more edges and flat, concave, or convex faces. The dosage forms may be symmetrical or asymmetrical, and may have features or geometries that allow for controlled, convenient, and easy storage, handling, packaging or dosing.

Oral transmucosal drug delivery is simple, non-invasive, and can be accomplished by a caregiver or patient with minimal discomfort. A dosage form for oral transmucosal delivery may be solid or non-solid. In one preferred embodiment, the dosage from is a solid that turns into a hydrogel following contact with saliva. In another preferred embodiment, the dosage from is a solid that erodes without forming a hydrogel following contact with saliva.

Generally, oral transmucosal delivery of pharmaceutically active substances is achieved using solid dosage forms such as lozenges or tablets, however, liquids, sprays, gels, gums, powders, and films and the like may also be used.

The claimed drug dosage forms are designed and adapted to deliver a substantial amount of drug to a subject via the oral mucosa.

Formulations for preparation of the claimed dosage forms and methods of making them are described in US Application Serial Nos. 11/825,251 and 11/650,227. An exemplary formulation is bioadhesive and comprises from about 0.0004% to about 0.04% sufentanil, e.g., 0.0005%, 0.001%, 0.002%, 0.003%, 0.004%, 0.006%, 0.008%, 0.01%, 0.012%, 0.014% or 0.016% sufentanil. In general, the formulation comprises(a) a non-ordered mixture of a pharmaceutically active amount of a drug; (b) a bioadhesive material which provides for adherence to the oral mucosa of the subject; and (c) stearic acid, wherein dissolution of a dosage form comprising the formulation is independent of pH, e.g., over a pH range of about 4 to 8.

Numerous suitable nontoxic pharmaceutically acceptable carriers for use in oral dosage forms can be found in Remington's Pharmaceutical Sciences, 17th Edition, 1985.

It will be understood that the formulation is converted into a dosage form for oral transmucosal administration to a subject using procedures routinely employed by those of skill in the art, such as direct compression, wet granulation, etc. The process for preparation of the dosage form is optimized for each formulation in order to achieve high dose content uniformity.

In a related approach, the combination of an opioid, such as a fentanyl congener and a benzodiazepine may be administered by inhalation or sublimation to sedate and provide analgesia to a subject during a diagnostic or therapeutic procedure or prior to induction of general anesthesia.

### Single Dose applicators

The invention further provides dispensing devices comprising the single small solid dosage form of the present invention for oral transmucosal delivery of a drug dosage form to a subject for procedural sedation and analgesia.

Application of a dispensing device or single dose applicator (SDA) for oral transmucosal delivery of a single small solid dosage form of the invention form for procedural sedation and analgesia is not limited to any particular type of device or patient population. As such, the SDAs find utility in drug delivery to pediatric, adult and non-human mammalian subjects.

The SDA is used to administer the single small solid dosage form of the invention.

The SDA may be provided as a pair of forceps, a syringe, a stick or rod, a straw, a pad, a capsule, a cup, a spoon, a strip, a tube, an applicator, a dropper, a patch, an adhesive pad, an adhesive film, a sprayer, an atomizer, or any other form suitable for the application of a single drug dosage form to the oral mucosa of a subject, e.g., the oral mucosa in the sublingual space. As will be understood by one of skill in the art, the SDA design may vary, so long as it is effective to place the tablet, in the desired location on an oral mucosal membrane, e.g., in the sublingual space, in a manner that preserves integrity of the drug dosage form in the dispensing process. After use, the SDA is disposed of, so as to eliminate the risk of contaminating the drug dispensing device with saliva, or other contaminants.

The SDA may contain the dosage form within, may have the drug dosage form attached or affixed to it, may have the dosage form dissolved in it, and may afford a seal against moisture, humidity, and light. The SDA may be manually manipulated by a patient, healthcare provider, or other user to place the dosage form in the proper location for drug delivery.

The single-dose applicator is used to deliver the small solid dosage form of the invention into the hand, the mouth, under the tongue, or to other locations appropriate for specific drug delivery needs.

In one embodiment, a single-dose applicator or drug dispensing device is used to deliver a dosage form to the oral mucosa, e.g., the sublingual space.

The dosage forms inside the SDA remain dry prior to dispensing, at which point a single dosage form is dispensed from the device into the mouth, e.g., the sublingual space, wherein a patient's saliva will wet the tablet and allow for tablet disintegration/erosion and drug dissolution. After use, the SDA is disposed of.

In one aspect of the invention, a small volume dosage form according to the present invention is placed in the sublingual cavity, preferably under the tongue on either side of the frenulum linguae, such that it adheres upon contact.

In one approach, for sublingual administration, a small volume dosage form may be administered by placement under the tongue, adjacent to the frenulum using forceps. Alternatively, a small volume dosage form may be administered by placement under the tongue, adjacent to the frenulum using a syringe, a syringe-type SDA, a stick or rod, a straw, a dropper, or any other form suitable for the application of a single drug dosage form, including but not limited to a SDA.

A plurality of SDAs may be provided as a series of individual SDAs attached by the backing or housed in a multiple dose dispenser or multiple dose storage unit.

In the case of procedural sedation and analgesia, the dispensing device is typically a SDA. In some cases, SDAs are stored in a multiple dose storage unit which may be referred to as a multiple dose applicator (MDA).

The dosage form may be provided in a package that consists of molded plastic or laminate that has indentations ("blisters") into which a dosage form is placed, referred to herein as a "blister pack". A cover, typically a laminated material or foil, is used to seal to the molded part. A blister pack may or may not have preformed or molded parts and may be used to package an SDA of any type.

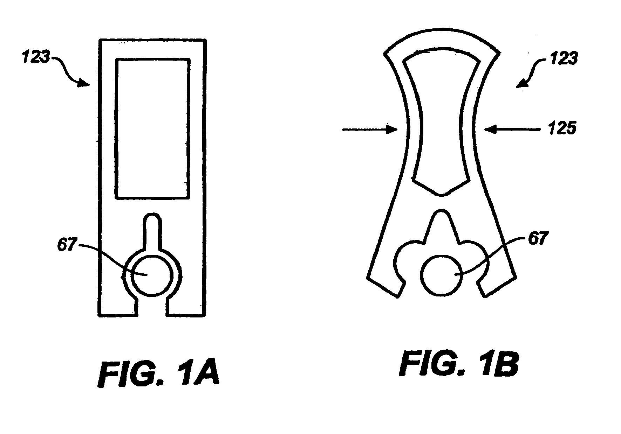

Figs. 1A-B, 2A-C, Figs. 3A-F, Figs. 5A-C, Figs. 6A-B, Figs. 7A-D, Fig. 8C and Figs. 9A-B are schematic depictions of exemplary SDAs for use in oral transmucosal administration of a drug dosage form.

Figs. 1A and 1B show one embodiment of a SDA **123** a dispensing device for delivering drug dosage forms. The dispensing device shown in Fig. 1A depicts the SDA **123** that is ready to dispense a drug dosage form **67.** In one aspect of this embodiment, a user pinches the SDA **123** which opens the applicator and a drug dosage form **67** is dispensed as shown in Fig. **1B****.**

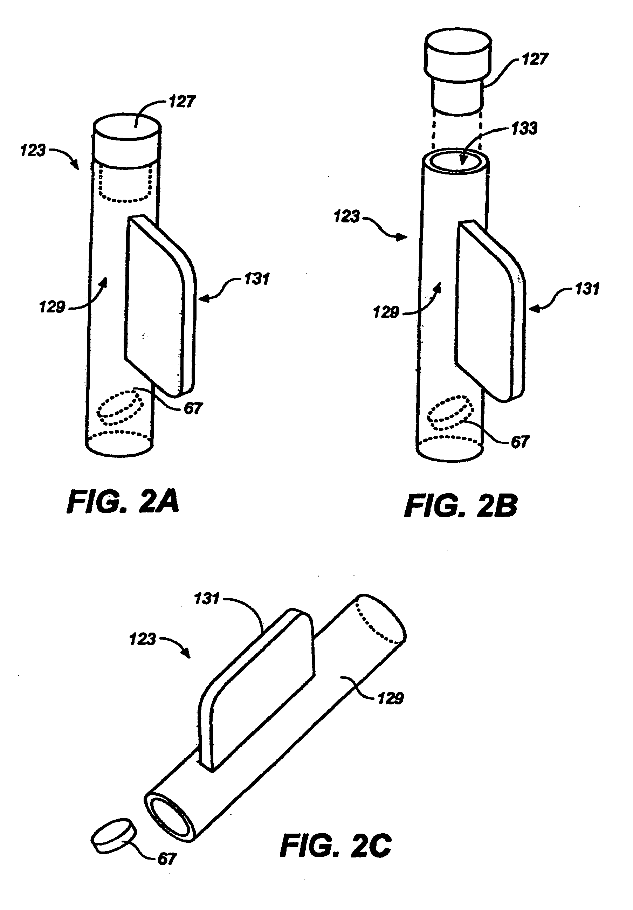

Figs. 2A - C show an embodiment of a SDA **123** that is comprised of a applicator shaped as a tube **129,** which has a stopper seal **127,** a handle **131** (e.g., an ergonomic handle), and a single dosage form **67.** Fig 2A shows the SDA **123** in its sealed configuration, prior to use. Fig 2B shows the SDA **123** with its stopper seal **127** removed, forming an opening **133,** and ready for use. Fig 2C shows the SDA **123** tilted so as to dispense the dosage form **67** on the oral mucosa, e.g., in the sublingual space.

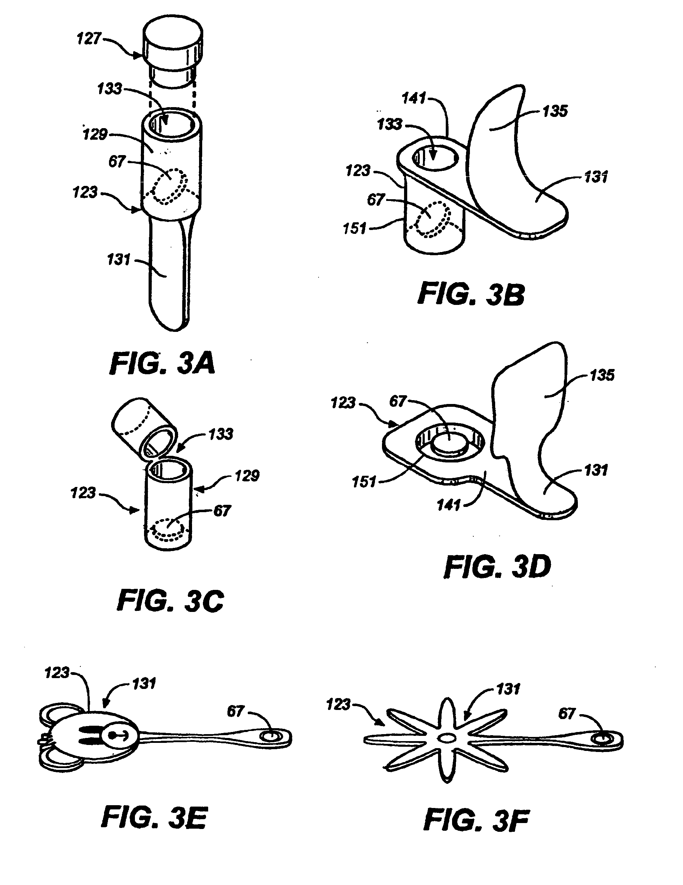

Figs. 3A - F show several alternate embodiments of the SDA **123.** In all of these figures the applicator seal **127** is broken and the applicator is tilted so as to drop the drug dosage form **67** adjacent an oral mucosal membrane in the mouth of a subject, e.g., under the tongue for sublingual dosage form placement. Fig. 3A shows a tube like applicator **129** with a handle **131 located** axially under the tube **129.** Fig. 3B shows an applicator formed as a thermoform or blister package **151** with a foil seal **135** that is peeled so as to open the applicator package **141** prior to placing the dosage form **67.** Fig. 3C shows an applicator that is a tube **129** which is broken to break the seal prior to dosage form **67** placement. Fig. 3D shows a blister pack tube **151** type dosage form package **141** with a handle **131** such that after the seal **135** is peeled back the blister pack 151 can be held and tilted to place the drug dosage form **67,** on an oral mucosal membrane. Figs. **3E** and **3F** show blister pack **151** type packaging with a handle **131** shaped like a flower or an animal, respectively, to be used for a SDA **123** designed for pediatric use. Other SDA shapes could include cartoon characters, animals, super-heroes or other appropriate shapes for pediatric applications.

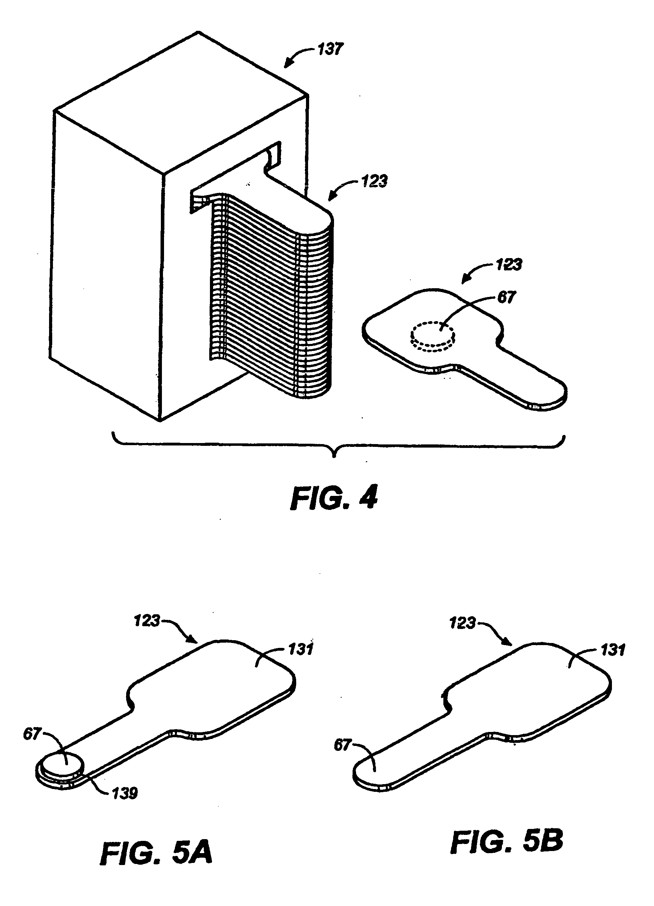

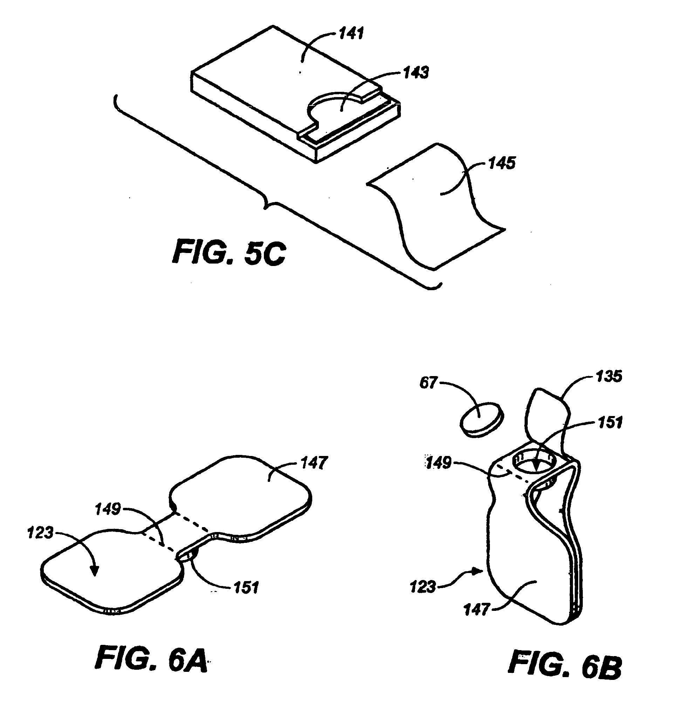

Figure 5A shows a flat rigid applicator **123** with a dosage form **67** adhered to one end, for example, by means of a rapidly dissolving ingestible adhesive material such that when the applicator end with the dosage form is placed under the tongue, the adhesive dissolves, the dosage form **67** is placed on an oral mucosal membrane, such as in the sublingual space, and the applicator can be removed. Fig 5B shows an applicator **123** made from a water permeable material, impregnated with drug, forming a material and dosage from matrix. When the impregnated end of this applicator **123** is placed under in the mouth on an oral mucosal membrane, the moisture in the saliva dissolves the drug and delivers it transmucosally. figure 5C shows dissolving film dosage forms **145** and a dosage form package with a plurality of dissolving film dosage forms **143** within it. The dissolving film dosage form **143** is removed from the package **141** and placed on an oral mucosal membrane, e.g., in the sublingual space where it dissolves and delivers the drug transmucosally.

Figs. 6A - B provides an illustration of two stages of use of one embodiment of a SDA **123.** Figure 6A shows the applicator **123** in its configuration prior to use, with two applicator tabs **147,** two perforations **149,** and a blister pack **151** containing a dosage form 67. In order to administer the dosage form **67,** the two applicator tabs **147** are bent downward at the perforations **149,** forming a handle **131** (Fig. 6B), and the seal **135** is peeled back to reveal the blister pack **151** and allow the dosage form **67** to be dropped on an oral mucosal membrane, e.g., in the sublingual space.

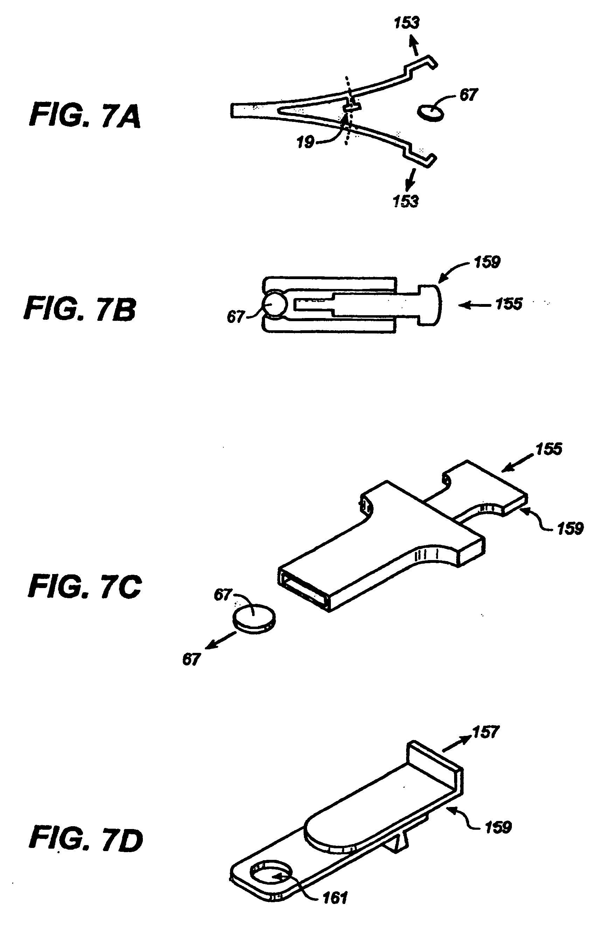

Figs. 7A - D are schematic depictions of additional examples of SDAs, including a tweezer or reverse scissor-type SDA (7A), where a drug dosage form **67** is held between the two sides **153** of the SDA **123** such that when the latch **19** is released, the drug dosage form **67** is no longer held by the SDA and can be placed on an oral mucosal membrane by the user; a syringe-type SDA (7B) with a circular channel, where a drug dosage form **67** is pushed out of the end of the channel when a user pushes **155,** the slider or plunger **159;** a pusher-type SDA (7C) with a rectangular channel where a drug dosage form **67** is pushed out of the end of the channel when a user pushes **155,** the slider **159;** or a slider-type SDA (7D) where drug dosage form **67** is held in a pocket **161** and the drug dosage form 67 becomes accessible when a user pulls **157** a slider **159.**

In another embodiment, a drug dispensing device of the invention may contain a plurality of SDAs, in a cartridge or individually packaged, and may dispense a single SDA containing a single drug dosage form for use by the patient, healthcare provider, or user. The drug dispensing device may dispense single SDAs in the same way and with the same features as would be advantageous for the dispensing of single drug dosage forms described in the invention. See e.g., Fig. 4 which is a schematic depiction of an exemplary multiple dose applicator **137** for delivering dispensing drug dosage forms **67,** each individually packaged in a SDA **123.**

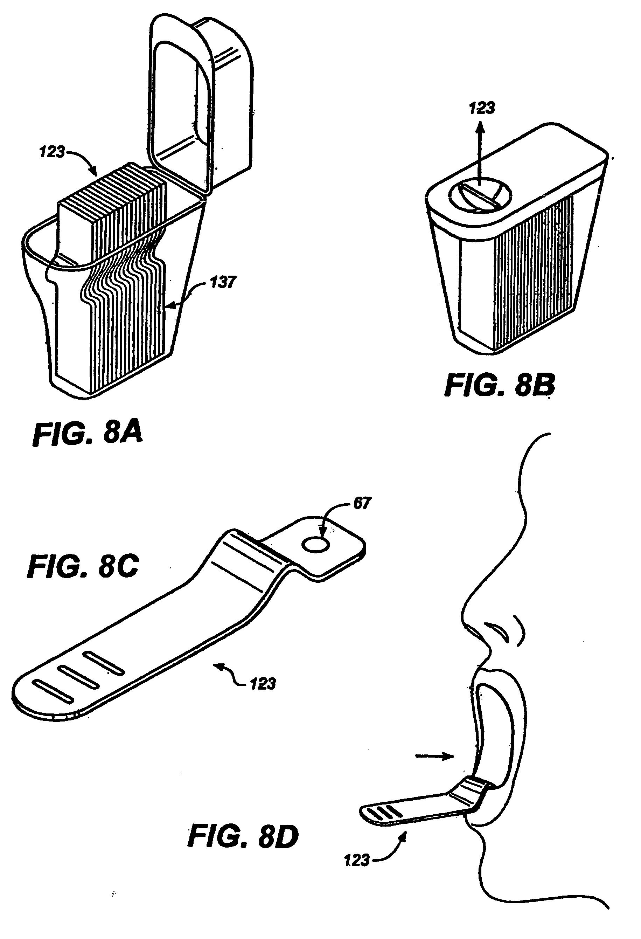

Figs. 8A - D provide a schematic depiction of a multiple dose applicator (MDA) **137** or container for storage of a plurality of SDAs **123** prior to use (8A); where in the exemplified embodiment, there is a slot in the upper cover of the MDA **137** for removal of individual SDAs **123** (8B); such that each individual SDA **123** comprises a drug dosage form **67** (8C); and the SDA **123** facilitates placement of the drug dosage form **67** under the tongue in the sublingual space (8D).

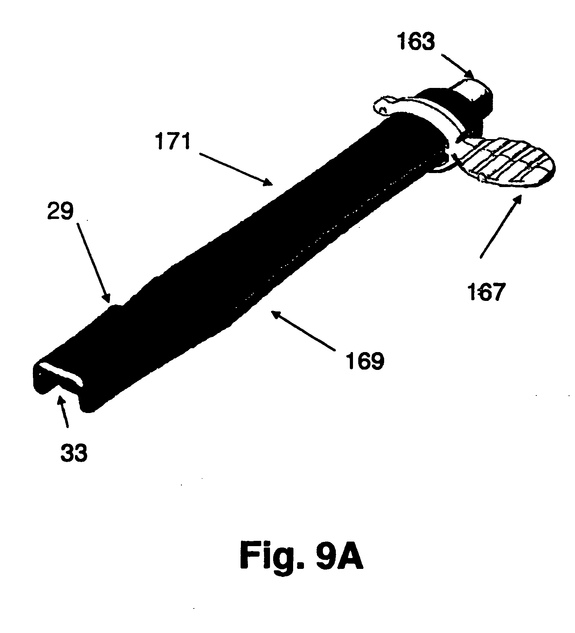

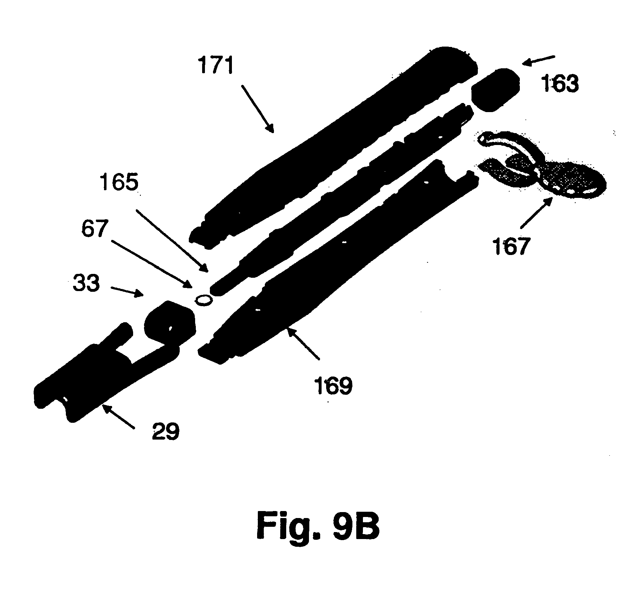

Fig. 9A is a schematic depiction of an embodiment of an SDA for delivery of an oral transmucosal dosage form to a subject. The SDA is provided in child resistant packaging as an individual SDA or housed in a multiple dispenser package (i.e., an MDA). The SDA has a pin lock **167** which serves as a lock-out feature and must be removed before a tablet can be injected from the SDA, as well as a pusher button **163,** which is pushed by a user to eject a tablet into the mouth of the subject on a mucosal membrane, e.g., adjacent the frenulum in the sublingual space. The SDA may be disassembled and has a bottom clamshell **169** and a top clamshell **171.** The SDA also has a shroud **29** and a valve **33,** which serve to protect the tablet from saliva ingress when the SDA is inserted into the mouth of a subject. Fig. 9B is an exploded view of a schematic depiction of the SDA shown in Fig. 9A wherein the bottom clamshell **169** and top clamshell **171** are separated illustrating the pusher **165** and a dosage form **67,** as well as the relative location of the dosage form **67,** the valve **33,** and the shroud **29.**

### Utility

Oral transmucosal drug delivery provides a simple, non-invasive means to administer a single drug dosage form in order to achieve sedation and analgesia. For certain drugs, such as those with poor bioavailability via the GI tract, and in situations where the patient cannot ingest an oral medication, such as prior to anesthesia, oral transmucosal delivery provides a significant advantage over traditional methods of oral administration, wherein the drug is swallowed.

The oral transmucosal dosage forms described herein find utility in delivery of a combination of sufentanil and triazolam for procedural sedation and analgesia. The small volume oral transmucosal dosage forms described herein provide for high relative AUC₀₋ₗₐₛₜ, low variability in Tₘₐₓ, low variability in Cₘₐₓ and low variability in AUC. The sedative effect of the drug combinations described herein may be the result of an additive or synergistic pharmacodynamic effect and/or may be due to the different onset and offset times of the opioid and benzodiazepine components of the combination.

Although benzodiazepines such as triazolam and midazolam have been used for procedural sedation, studies have shown that transient residual amnesia frequently occurs when oral doses of benzodiazepines above 250 mcg are administered. Although triazolam is a short-acting benzodiazepine, it may still cause residual impairment into the next day, with "hangover" effects such as sleepiness, impaired psychomotor and cognitive functions, any of which can impair the ability of users to drive safely, etc. (Vermeeren A., 2004, CNS Drugs. 18 (5): 297-328). It has also been shown that benzodiazepines such as triazolam cause a greater degree of sedation and greater impairment of psychomotor performance in healthy elderly persons than in young persons who received the same dose (Greenblatt et al., 1991). Therefore it is important to minimize the dose of this agent, for example by adding another agent, such as sufentanil, which can enhance the sedative properties of triazolam.

The results described herein show that oral transmucosal delivery of the combination of sufentanil and triazolam were effective to sedate alert, awake subjects in a human clinical trial. The amount of sedation as measured by the total AUC of the RASS sedation score was greater for the combination of sufentanil and triazolam, than for the equivalent dose of sufentanil alone. Hence, the combination of sufentanil and triazolam produced a higher degree of sedation than sufentanil alone, yet the duration of analgesia was not prolonged, being approximately 4 hours for both treatments.

The claimed drug combinations find particular utility in pediatric applications, since the comfortable and secure nature of the dosage form will allow small children to readily accept this mode of therapy and will reliably deliver drug transmucosally. Specific examples include, but are not limited to, sedation and analgesia associated with a medical or dental procedure or in an emergency situation, in particular, when IV access is not available or inconvenient, when a child is NPO (no oral intake allowed) or when rapid onset of drug effect is required.

The dosage forms of the invention find further utility in veterinary applications.

All publications mentioned herein are referenced for the purpose of describing and disclosing the compositions and methodologies which are described in the publications which might be used in connection with the presently described invention. The publications discussed herein are provided solely for their disclosure prior to the filing date of the present application. Nothing herein is to be construed as an admission that the invention is not entitled to antedate such a disclosure by virtue of prior invention.

The following examples are provided to illustrate the invention and are not intended to limit any aspect of the invention as set forth above or in the claims below.

### EXAMPLES

### EXAMPLE 1. Phase 1 Clinical Study of Sublingual Sufentanil

Two different sublingual sufentanil formulations were previously evaluated in a Phase 1 clinical trial, including a slower-eroding form (erosion time of approximately 15-25 minutes), and a faster-eroding form (approximate erosion time of 6-12 minutes). Patients were blocked with a mu-opioid receptor antagonist, naltrexone (50 mg orally twice per day). In a study detailed in U.S. Application Serial No. 11/985,162, sufentanil plasma concentrations with respect to time were analyzed and tabulated. The maximum sufentanil concentration in plasma (Cₘₐₓ), time to Cₘₐₓ (Tₘₐₓ), area under the curve (AUC_{inf}), F and terminal t_{1/2} including Cₘₐₓ, Tₘₐₓ, and t_{1/2} were evaluated for each dose group. The relevant results are summarized below in Tables 1A and 1B.
**EXAMPLE 1A:** All subjects received a 10-minute IV infusion of 5 mcg sufentanil. After a 1-day washout period, each subject then received a single sublingual administration of a dosage form (comprising a slow-eroding formulation) containing 2.5 mcg of sufentanil. On the two subsequent study days, the dose was escalated, and each subject received a dosage form (comprising a slow-eroding formulation) containing 5 and 10 mcg of sufentanil.
**EXAMPLE 1B:** All subjects received four repeated sublingual doses of a dosage form (comprising a slow-eroding formulation) containing 5 mcg of sufentanil administered at 10-minute intervals.

Table 1A provides a summary of pharmacokinetic parameters including Cₘₐₓ, Tₘₐₓ, AUC_{inf}, F and t_{1/2}. The Cₘₐₓ after multiple sublingual dosing was 46.36 pg/mL The mean AUC_{inf} increased with multiple sublingual dosing of sufentanil and was generally proportional to dose when compared to single sublingual administration.

**Table 1A. Summary of Sufentanil Pharmacokinetic Parameters**

| Parameter | | 5 mcg IV | 2.5 mcg | 5 mcg | 10 mcg | 4x5 mcg |
|---|---|---|---|---|---|---|
| Cₘₐₓ | (pg/mL) | 81.3± 28.1 | 6.8± 2.1 | 10.9± 3.5 | 27.5± 7.7 | 46.4±12.4 |
| Tₘₐₓ | (hr) | 0.16± 0.03 | 0.73± 0.13 | 0.77±0.29 | 0.68±0.22 | 1.16± 0.23 |
| AUC_{inf} | (hr*pg/mL) | 38.4± 8.5 | 18.0± 4.5 | 27.4± 9.1 | 71.2± 20.7 | 146.5± 39.1 |
| t_{1/2} | (hr) | 1.66± 0.72 | 1.71± 0.51 | 1.56± 0.57 | 1.97±0.85 | 3.29± 1.10 |
| F | (%) | - | 95.3± 19.1* | 74.5± 26.3* | 95.5± 29.2* | 97.2± 21.2* |

| | | | | | | |
|---|---|---|---|---|---|---|
| * %F calculated using 5mcg IV AUC | | | | | | |

**EXAMPLE 1C:** Subjects were administered a 10-minute IV infusion of 5mcg sufentanil, a single sublingual administration of a dosage form containing 10mcg of sufentanil (faster-eroding formulation) and four repeated sublingual doses of a dosage form containing 10mcg of sufentanil (faster-eroding formulation) administered at 20-minute intervals.
**EXAMPLE 1D:** Subjects were administered a 20-minute IV infusion of 50mcg sufentanil and a single sublingual administration of a dosage form containing 80mcg of sufentanil (faster-eroding formulation).

Table 1B provides a summary of pharmacokinetic parameters including Cₘₐₓ Tₘₐₓ, AUC_{inf}, F and t_{1/2}.

**Table 1B. Summary of Sufentanil Pharmacokinetic Parameters**

| **Parameter** | | **5 mcq IV** | **10mcg** | **4x10mcg** | **80mcg** | **50mcg IV** |
|---|---|---|---|---|---|---|
| **Cₘₐₓ** | (pg/mL) | 63.9± 28.2 | 16.5± 6.8 | 78.7± 20.1 | 127.2± 42.3 | 561.1± 277.7 |
| **Tₘₐₓ** | (hr) | 0.17± 0.0 | 0.84± 0.35 | 1.41± 0.25 | 0.89± 0.35 | 0.34± 0.11 |
| **AUC_{inf}** | (hr*pg/mL) | 39.4± 9.6 | 44.9± 24.6 | 253.4 ± 70.1 | 382.1± 88.2 | 528.0± 134.4 |
| **t_{1/2}** | (hr) | 1.72± 0.47 | 1.67± 0.67 | 3.54± 1.02 | 4.23± 0.90 | 3.69± 0.78 |
| **F** | (%) | - | 60.9± 27.7* | 87.8± 22.2* | 70.1± 20.1* | - |

| | | | | | | |
|---|---|---|---|---|---|---|
| * %F calculated using 5mcg IV AUC | | | | | | |

### EXAMPLE 2. Phase 1 Clinical Study of Sublingual Sufentanil and Triazolam for Procedural Sedation and Analgesia

The pharmacokinetics and pharmacodynamics of sufentanil and/or triazolam administered via the sublingual route using a tablet of 3 different strengths were evaluated in a Phase 1 clinical trial. The experimental design is a randomized 2 cohort, 5-arm crossover, open-label on days 1 and 2, double-blinded on days 3 to 5, single-dose, fasting design. The study involved 24 normal, healthy, non-smoking male and female subjects, divided into 2 cohorts as follows: Cohort 1: 12 male and female subjects within the age range of 18 and 60 years and Cohort 2: 12 male and female subjects within the age range of 61 and 80 years.

The study relied on a single 7-day study period for each subject and each cohort received: Day 1: Halcion^{®} (triazolam) 125 mcg tablets orally; Day 2: 5 mcg sufentanil IV (slow infusion). Cohort 1 also received a sublingual tablet containing 10 mcg of sufentanil and 200 mcg of triazolam, 15 mcg of sufentanil and 200 mcg of triazolam or 10 mcg of sufentanil alone on days 3-5 in a randomized, blinded design. Cohort 2 also received a sublingual tablet containing 10 mcg of sufentanil and 200 mcg of triazolam, 10 mcg of sufentanil and 100 mcg of triazolam or 10 mcg of sufentanil alone on days 3-5 in a randomized, blinded design. The fractional (%) compositions of the formulation for each dosage form/tablet of sufentanil and triazolam are shown in Table 2.

**Table 2: Fractional Composition Per Tablet Of Sufentanil/Triazolam.**

| **Ingredient** | **10 mcg sufentanil/100 mcg triazolam (%w/w)** | **10 mcg sufentanil/200 mcg triazolam (%w/w)** | **15 mcg sufentanil/200 mcg triazolam (%w/w)** |
|---|---|---|---|
| Sufentanil Citrate, USP | 0.256 | 0.256 | 0.385 |
| Triazolam, (Conforms to USP) | 1.709 | 3.419 | 3.419 |
| Mannitol, EP/USP/JP | 68.784 | 67.075 | 66.947 |
| Dicalcium Phosphate Dihydrate, USP/FCC/EP | 20.000 | 20.000 | 20.000 |
| Hydroxypropyl Methylcellulose (Methocel) K4M Premium CR, EP | 3.000 | 3.000 | 3.000 |
| Stearic Acid, NF/EP/BP/JP | 5.000 | 5.000 | 5.000 |
| Magnesium Stearate, NF | 1.000 | 1.000 | 1.000 |
| Butylated Hydroxytoluene,USP | 0.250 | 0.250 | 0.250 |
| **Total** | **100.00** | **100.00** | **100.00** |

The mass (in mg) composition per tablet for each strength of sufentanil and triazolam tablets is shown in Table 3.

**Table 3: Mass (mg) Composition per Tablet for each Strength of Sufentanil/Triazolam Tablets.**

| **Ingredient** | **10 mcg sufentanil/100 mcg triazolam (mg/tablet)** | **10 mcg sufentanil/200 mcg triazolam (mg/tablet)** | **15 mcg sufentanil/200 mcg triazolam (mg/tablet)** |
|---|---|---|---|
| Sufentanil Citrate, USP | 0.015 | 0.015 | 0.0225 |
| Triazolam (conforms to USP) | 0.1000 | 0.2000 | 0.200 |
| Mannitol, EP/USP/JP | 4.024 | 3.924 | 3.916 |
| Dicalcium Phosphate Dihydrate, USP/FCC/EP | 1.170 | 1.170 | 1.170 |
| Hydroxypropyl Methylcellulose (Methocel) K4M Premium CR, EP | 0.176 | 0.176 | 0.176 |
| Stearic Acid, NF/EP/BP/JP | 0.293 | 0.293 | 0.293 |
| Maqnesium Stearate, NF | 0.059 | 0.059 | 0.059 |
| Butylated Hydroxytoluene,USP | 0.0146 | 0.0146 | 0.0146 |
| **Total (mg)** | **5.85** | **5.85** | **5.85** |

The fractional (%) and mass (mg) composition for the 10 mcg strength of sufentanil tablets are shown in Table 4.

**Table 4. Fractional and Mass Composition of the 10 mcg Sufentanil Tablets.**

| **Ingredient** | **10 mcg sufentanil** (%w/w) | **10 mcg sufentanil** (mg/tablet) |
|---|---|---|
| Sufentanil Citrate, USP | 0.256 | 0.015 |
| Mannitol, EP/USP/JP | 74.9 | 4.122 |
| Dicalcium Phosphate Dihydrate, USP/FCC/EP | 20.000 | 1.170 |
| Hydroxypropyl Methylcellulose (Methocel) K4M Premium CR, EP | 3.000 | 0.176 |
| Stearic Acid, NF/EP/BP/JP | 5.000 | 0.293 |
| Maqnesium Stearate, NF | 1.000 | 0.059 |
| Butylated Hydroxytoluene,USP | 0.250 | 0.0146 |
| **Total** | **100.00** | **5.85** |

A series of blood samples were drawn during the study as exemplified by the following schedule: On days 1 to 5: One sample was drawn prior to dosing and at approximately 5, 10, 15, 20, 40, 60, 90, 120, 160, 240, 320, 480, and 640 minutes post-dosing.

Pharmacokinetic (PK) parameters, including the following, were calculated for sufentanil and triazolam: AUC₀₋ₗₐₛₜ, Cₘₐₓ, Tₘₐₓ, t_{½} and relative AUC₀₋ₗₐₛₜ.

Analysis of sufentanil and triazolam was carried out according to the following method. Sufentanil, triazolam and internal standards fentanyl and triazolam-D4 were extracted from 0.2 ml human plasma by solid phase extraction into an organic medium and reconstituted in 200mcl of reconstitution solution. An aliquot was injected into a High Performance Liquid Chromatography system and detected using a TSQ Quantum tandem mass spectrometer and quantitated using a peak ratio method. Analyses of sufentanil and triazolam were conducted at Biovail Contract Research.

Pharmacodynamic (PD) parameters were evaluated using sedative scores [+4 to -5 for the RASS, and 0 to 10 for the Numeric Rating Scale (NRS). The RASS score and NRS score were determined and recorded for each patient at a number of time-points after each dose. The RASS is used as a substantially objective assessment for sedation and includes a scale from -5 (unarousable) to +4 (combative), and includes a procedure on assessing and assigning the sedation score for a patient. The Numeric Rating Scale (NRS) provides for a subject's own assessment of sedation using an 11-point NRS, where patients were asked their level of sedation of a scale of 0 to 10, where 0 = feeling awake and alert, and 10 =asleep.

No adverse events related to nausea/vomiting or respiratory sedation occurred during this study for any subject with any dose of study medication.

The results of an analysis of onset of RASS Sedation (hours) in subjects who were less than 61 years old are shown in Table 5.

**Table 5. Analysis of Onset of RASS Sedation (hours) Subjects Who Were Less Than 61 Years Old**

| **N** | **12** | **12** | **9** |
|---|---|---|---|
| | Sufentanil 10/ Triazolam 200 (mcg) | Sufentanil 15/ Triazolam 200 (mcg) | Sufentanil 10mcg |
| **Mean** | 0.841 | 0.584 | 0.964 |
| **(SD)** | 0.69 | 0.352 | 0.908 |

The results of an analysis of total AUCof RASS sedation in subjects who were less than 61 years old are shown in Table 6.

**Table 6. Analysis of AUCₜₒₜₐₗ of RASS Sedation: Subjects Who Were Less Than 61 Years Old.**

| **N** | **12** | **12** | **9** |
|---|---|---|---|
| | Sufentanil 10/ Triazolam 200 (mcg) | Sufentanil 15/ Triazolam 200 (mcg) | Sufentanil 10mcg |
| **RASS Mean** | 7.537 | 8.116 | 4.259 |
| **RASS (SD)** | 3.939 | 4.435 | 3.252 |

The results of an analysis of total duration of RASS sedation in subjects who were less than 61 years old are shown in Table 7.

**Table 7. Analysis of AUC of Total Duration of RASS Sedation: Subjects Who Were Less Than 61 Years Old**

| **N** | **12** | **12** | **9** |
|---|---|---|---|
| | Sufentanil 10/ Triazolam 200 (mcg) | Sufentanil 15/ Triazolam 200 (mcg) | Sufentanil 10 mcg |
| **RASS Mean** | 4.048 | 3.972 | 2.843 |
| **RASS (SD)** | 1.486 | 1.839 | 2.388 |

The results of an analysis of onset of RASS Sedation (hours) in subjects who were at least 61 years old is shown in Table 8.

**Table 8. Analysis of Onset of RASS Sedation (hours) Subjects Who Were At Least 61 Years Old**

| **N** | **9** | **9** | **10** |
|---|---|---|---|
| | Sufentanil 10/ Triazolam 200 (mcg) | Sufentanil 10 mcg | Sufentanil 10/ Triazolam 100 (mcg) |
| **Mean** | 0.446 | 0.436 | 0.343 |
| **(SD)** | 0.34 | 0.129 | 0.167 |

The results of an analysis of total AUC of RASS sedatior at least 61 years old are shown in Table 9.

**Table 9. Analysis of AUCₜₒₜₐₗ of RASS Sedation Assessment: Subjects Who Were At Least 61 Years Old**

| **N** | **12** | **12** | **12** |
|---|---|---|---|
| | Sufentanil 10/ Triazolam 200 (mcg) | Sufentanil 10 mcg | Sufentanil 10/ Triazolam 100 (mcg) |
| **RASS Mean** | 9.732 | 5.203 | 6.724 |
| **RASS (SD)** | 8.501 | 4.651 | 4.866 |

The results of an analysis of total duration of RASS sedation in subjects who were at least 61 years old are shown in Table 10.

**Table 10. Analysis of AUC of Total Duration of RASS Sedation: Subjects Who Were At Least 61 Years Old**

| **N** | **12** | **12** | **12** |
|---|---|---|---|
| | Sufentanil 10/ Triazolam 200 (mcg) | Sufentanil 10 mcg | Sufentanil 10/ Triazolam 100 (mcg) |
| **RASS Mean** | 4.388 | 4.618 | 5.548 |
| **RASS (SD)** | 3.533 | 3.601 | 3.293 |

The results of pharmacokinetic analysis for sufentanil in subjects who were less than 61 years old are shown in Table 11.

**Table 11. Summary of Sufentanil Pharmacokinetic Parameters Subjects Who Were Less Than 61 Years Old**

| Parameter | | Sufentanil 10/ Triazolam 200 (mcg) | Sufentanil 15/ Triazolam 200 (mcg) | Sufentanil 10 mcg |
|---|---|---|---|---|
| Cₘₐₓ | (pg/mL) | 21.64 +/- 6.59 | 33.00 +/- 15.17 | 20.0 +/- 5.87 |
| Tₘₐₓ | (hr) | 0.94 +/- 0.39 | 0.82 +/- 0.17 | 0.74 +/- 0.28 |
| AUC₀₋ₗₐₛₜ | (hr*pg/mL) | 43.30 +/- 19.36 | 75.88 +/- 41.35 | 35:68 +/- 10.60 |
| t_{1/2} | (hr) | 4.65 +/- 3.40 | 2.64 +/- 0.78 | 3.37 +/- 1.60 |
| Relative AUC₀₋ₗₐₛₜ | (%) | 95% | 101% | 84% |

| | | | | |
|---|---|---|---|---|
| Data reported as mean +/- SD. Relative AUC₀₋ₗₐₛₜ values were obtained by normalizing the doses to the 5 mcg IV sufentanil comparator. | | | | |

The results of pharmacokinetic analysis for triazolam in subjects who were less than 61 years old is shown in Table 12.

**Table 12. Summary of Triazolam Pharmacokinetic Parameters Subjects Who Were Less Than 61 Years Old**

| Parameter | | Triazolam 125 mcg | Sufentanil 10/ Triazolam 200 (mcg) | Sufentanil 15/ Triazolam 200 (mcg) |
|---|---|---|---|---|
| Cₘₐₓ | (pg/mL) | 1224.8 +/- 385.0 | 1528.9 +/- 520.6 | 1553.5 +/- 448.0 |
| Tₘₐₓ | (hr) | 0.94 +/- 0.46 | 2.54 +/- 1.43 | 1.86 +/- 0.99 |
| AUC₀₋ₗₐₛₜ | (hr*pg/mL) | 5151.9 +/- 2364.7 | 9451.1 +/- 3721.4 | 9501.8 +/- 3639.3 |
| t_{1/2} | (hr) | 3.10 +/- 1.27 | 3.46 +/- 1.03 | 4.08 +/- 2.45 |
| Relative AUC₀₋ₗₐₛₜ | (%) | NA | 120% | 121% |

| | | | | |
|---|---|---|---|---|
| Data reported as mean +/- SD Relative AUC₀₋ₗₐₛₜ values were obtained by normalizing the doses to the 125 mcg oral triazolam comparator. | | | | |

The results of pharmacokinetic analysis for sufentanil in subjects who were at least 61 years old is shown in Table 13.

**Table 13. Summary of Sufentanil Pharmacokinetic Parameters Subjects Who Were At Least 61 Years Old**

| Parameter | | Sufentanil 10/ Triazolam 200 (mcg) | Sufentanil 10 mcg | Sufentanil 10/ Triazolam 100 (mcg) |
|---|---|---|---|---|
| Cₘₐₓ | (pg/mL) | 21.83 +/- 11.50 | 24.83 +/- 16.33 | 25.33 +/- 6.49 |
| Tₘₐₓ | (hr) | 1.00 +/- 0.29 | 0.88 +/- 0.48 | 0.75 +/- 0.21 |
| AUC₀₋ₗₐₛₜ | (hr*pg/mL) | 53.65 +/- 49.04 | 47.65 +/- 26.84 | 52.26 +/- 17.69 |
| t_{1/2} | (hr) | 5.32 +/- 5.20 | 5.02 +/- 6.32 | 3.65 +/- 2.44 |
| Relative AUC₀₋ₗₐₛₜ | (%) | 107% | 87% | 108% |

| | | | | |
|---|---|---|---|---|
| Data reported as mean +/- SD Relative AUC₀₋ₗₐₛₜ values were obtained by normalizing the doses to the 5 mcg IV sufentanil comparator. | | | | |

The results of pharmacokinetic analysis for triazolam in subjects who were at least 61 years old is shown in Table 14.

**Table 14. Summary of Triazolam Pharmacokinetic Parameters Subjects Who Were At Least 61 Years Old**

| Parameter | | Triazolam 125 mcg | Sufentanil 10/ Triazolam 200 (mcg) | Sufentanil 10/ Triazolam 100 (mcg) |
|---|---|---|---|---|
| Cₘₐₓ | (pg/mL) | 1139.8 +/- 490.3 | 1599.7 +/- 554.3 | 947.2 +/- 351.6 |
| Tₘₐₓ | (hr) | 0.97 +/- 0.45 | 2.53 +/- 1.19 | 2.22 +/- 1.45 |
| AUC₀₋ₗₐₛₜ | (hr*pg/mL) | 5437.2 +/- 3441.5 | 10867.1 +/- 5566.5 | 6007.2 +/- 3372.3 |
| t_{1/2} | (hr) | 3.46 +/- 1.23 | 4.66 +/- 2.21 | 4.45 +/- 1.79 |
| Relative AUC₀₋ₗₐₛₜ | (%) | NA | 132% | 140% |

| | | | | |
|---|---|---|---|---|
| Data reported as mean +/- SD Relative AUC₀₋ₗₐₛₜ values were obtained by normalizing the doses to the 125 mcg oral triazolam comparator. | | | | |

Although the foregoing has been described in some detail by way of illustration and example for purposes of clarity and understanding, it will be apparent to those skilled in the art that certain changes and modifications may be practiced. Various aspects of the invention have been achieved by a series of experiments, some of which are described by way of the following non-limiting examples. Therefore, the description and examples should not be construed as limiting the scope of the invention, which is delineated by the appended description of exemplary embodiments.

## Claims

1. A single small volume solid dosage form for use in procedural sedation and analgesia:
comprising the combination of from 5 micrograms (µg, mcg) to 50 µg (mcg) of sufentanil and from 100 µg (mcg) to 500 µg (mcg) of triazolam, wherein said solid dosage form is bioadhesive, and adapted to adhere to the oral mucosa until most or all of the drug has been delivered by oral transmucosal administration during the period of drug delivery, wherein said solid dosage form has a volume of less then 100 µl (mcl) and a mass of less than 100 mg.

2. The solid dosage form for use in procedural sedation and analgesia according to Claim 1, wherein said dosage form has a mass selected from the group consisting of less than 90mg, 80mg, 70mg, 60mg, 50mg, 40mg, 30mg, 29mg, 28mg, 27mg, 26mg, 25mg, 24mg, 23mg, 22mg, 21mg, 20mg, 19mg, 18mg, 17mg, 16mg, 15mg, 14mg, 13mg, 12mg, 11mg, 10mg, 9mg, 8mg, 7mg, 6mg and 5mg.

3. The drug dosage form for use in procedural sedation and analgesia according to Claim 1, wherein said oral transmucosal administration is sublingual administration.

4. The solid dosage form for use in procedural sedation and analgesia according to Claim 1, wherein following administration to said subject, onset of sedation is occurs in less than one hour.

5. The solid dosage form for use in procedural sedation and analgesia according to Claim 1, wherein following administration the duration of sedation is 4 hours or less.

6. A single dose applicator (SDA) for use in procedural sedation and analgesia, comprising a dosage form according to any one of Claim 1.

## Patentansprüche

1. Einzelne kleinvolumige feste Dosierungsform zur Verwendung bei prozeduraler Sedierung und Analgesie:
beinhaltend die Kombination von 5 Mikrogramm (µg, mcg) bis 50 µg (mcg) von Sufentanil und von 100 µg (mcg) bis 500 µg (mcg) von Triazolam, wobei die feste Dosierungsform bioadhäsiv ist, und angepasst ist, um an der oralen Mukosa zu haften, bis der Großteil oder das gesamte Arzneimittel während der Arzneimittelzuführung durch orale transmukosale Verabreichung zugeführt wurde, wobei die feste Dosierungsform ein Volumen von weniger als 100 µl (mcl) und eine Masse von weniger als 100 mg besitzt.

2. Feste Dosierungsform zur Verwendung bei prozeduraler Sedierung und Analgesie gemäß Anspruch 1, wobei die Dosierungsform eine Masse besitzt, ausgewählt aus der Gruppe bestehend aus weniger als 90 mg, 80 mg, 70 mg, 60 mg, 50 mg, 40 mg, 30 mg, 29 mg, 28 mg, 27 mg, 26 mg, 25 mg, 24 mg, 23 mg, 22 mg, 21 mg, 20 mg, 19 mg, 18 mg, 17 mg, 16 mg, 15 mg, 14 mg, 13 mg, 12 mg, 11 mg, 10 mg, 9 mg, 8 mg, 7 mg, 6 mg und 5 mg.

3. Dosierungsform zur Verwendung bei prozeduraler Sedierung und Analgesie gemäß Anspruch 1, wobei die orale transmukosale Verabreichung sublinguale Verabreichung ist.

4. Feste Dosierungsform zur Verwendung bei prozeduraler Sedierung und Analgesie gemäß Anspruch 1, wobei nach Verabreichung an das Subjekt, Einsetzen der Sedierung in weniger als einer Stunde auftritt.

5. Feste Dosierungsform zur Verwendung bei prozeduraler Sedierung und Analgesie gemäß Anspruch 1, wobei nach Verabreichung die Dauer der Sedierung 4 Stunden oder weniger ist.

6. Einzeldosisapplikator (SDA, single dose applicator) zur Verwendung bei prozeduraler Sedierung und Analgesie, beinhaltend eine Dosierungsform gemäß einer von Anspruch 1.

## Revendications

1. Une forme galénique solide individuelle de faible volume destinée à une utilisation dans une analgésie et une sédation procédurales :
comprenant la combinaison de 5 microgrammes (µg, mcg) à 50 µg (mcg) de sufentanil et de 100 µg (mcg) à 500 µg (mcg) de triazolam, où ladite forme galénique solide est bioadhésive et adaptée de façon à adhérer à la muqueuse buccale jusqu'à ce que la plus large part ou la totalité du médicament ait été alimentée par administration transmucosale orale au cours de la période de alimentation de médicament, où ladite forme galénique solide possède un volume inférieur à 100 µl (mcl) et une masse inférieure à 100 mg.

2. La forme galénique solide destinée à une utilisation dans une analgésie et une sédation procédurales selon la Revendication 1, où ladite forme galénique possède une masse sélectionnée dans le groupe se composant de moins de 90 mg, 80 mg, 70 mg, 60 mg, 50 mg, 40 mg, 30 mg, 29 mg, 28 mg, 27 mg, 26 mg, 25 mg, 24 mg, 23 mg, 22 mg, 21 mg, 20 mg, 19 mg, 18 mg, 17 mg, 16 mg, 15 mg, 14 mg, 13 mg, 12 mg, 11 mg, 10 mg, 9 mg, 8 mg, 7 mg, 6 mg et 5 mg.

3. La forme galénique de médicament destinée à une utilisation dans une analgésie et une sédation procédurales selon la Revendication 1, où ladite administration transmucosale orale est une administration sublinguale.

4. La forme galénique solide destinée à une utilisation dans une analgésie et une sédation procédurales selon la Revendication 1, où suite à une administration audit sujet, l'apparition de la sédation se produit en moins d'une heure.

5. La forme galénique solide destinée à une utilisation dans une analgésie et une sédation procédurales selon la Revendication 1, où suite à une administration, la durée de sédation est de 4 heures ou moins.

6. Un applicateur de dose individuelle (SDA) destiné à une utilisation dans une analgésie et une sédation procédurales comprenant une forme galénique selon la Revendication 1.
